# EUROPEAN PATENT APPLICATION

(11) **EP 1 286 280 A1**
(43) Date of publication of application: **26.02.2003**
(21) Application number: 00929891.0
(22) Date of filing: 26.05.2000
(51) Int. Cl.: G06F 17/60, G06F 19/00

(54) **MEDICAL DATA PROCESSING SYSTEM AND RECORDING MEDIUM FOR DATA PROCESSING SYSTEM**

(71) Applicant: Kabushiki Kaisha TOPCON, Tokyo 174-0052 (JP)
(72) Inventor: NADACHI, Ryoichi, c/o Kabushiki Kaisha Topcon, Tokyo 174-0052 (JP); KATO, Takeyuki, c/oKabushiki Kaisha Topcon, Tokyo 174-0052 (JP)
(74) Representative: Pfenning, Meinig & Partner
(86) International application number: JP0003419
(87) International publication number: WO01090976

(57) **Abstract**

A medical information processing system and a medical information processing recording medium therefor. In the medical information processing system, main control circuits are set to check databases of data recording/reproducing means at a specified time interval or at a specified time. If the databases contain new patient information, the main control circuits automatically prepare e-mail including the new patient information and the patient's ID and send the e-mail through the Internet to one or more predetermined destinations such as ophthalmology data processing terminals.

## Description

### BACKGROUND OF THE INVENTION

### 1. Field of the Invention

This invention relates to a medical information processing system for transmitting and receiving medical data among two or more medical information processing apparatuses through a network, and to a recording medium for the information processing system.

### 2. Background Art

In recent years, as information transmitting means has been developed, conceived is a medical information processing system for transmitting and receiving data among medical information processing apparatuses operated in two and more spots by means of network connection using a dedicated line. For example, as such a medical information processing system, conceived is an ophthalmologic information processing system for transmitting and receiving ophthalmologic data among medical information processing apparatuses mainly operated in two and more remote places.

Meanwhile, as inexpensive communication means, the Internet using a general communication line is also utilized. In the foregoing ophthalmologic information processing system, when image data and a patient accompanying with it and image information are shared or copied among the information processing apparatuses in two or more places, heretofore, the Internet has been utilized as inexpensive communication means.

When images received by e-mail are added with graphic information and observation/diagnosis information and sent back to a sender, an address book has been heretofore referred to separately at each sending back based on patient information of the images, and thus a destination to be replied to has been designated.

However, when the images and the accompanying patient and image information are to be shared or copied among a plurality of ophthalmologic image information systems by use of the Internet connected through a telephone line, if the image data and the accompanying patient and image information are only attached to e-mail and sent as has been done heretofore, an operator him/herself has had to call out such information from a database at each sharing and copying, and to prepare and transmit e-mail having the information attached thereto. On the other hand, also on an e-mail receiving side, an operation in which an operator him/herself separates such information from the e-mail and stores the information in a database has been necessary.

Moreover, when the graphic information and the observation/diagnosis information are added to the images sent by e-mail and sent back to a sender, the address book has heretofore been separately referred to at each sending back based on the patient information of the images, and thus the address to be replied has been designated. However, a procedure thereof has been cumbersome, causing a problem such as a mistake in designating the address to be replied and a wrong address.

Accordingly, a first object of the present invention is to provide a medical information processing system capable of automatically preparing e-mail including new patient information and of automatically transmitting the e-mail to destination medical information processing apparatuses when the new patient information is newly added and stored in a database.

Moreover, a second object of the present invention is to provide a recording medium for an information processing system, the recording medium having a program capable of automatically preparing e-mail including new patient information and of automatically transmitting the e-mail to destination medical information processing apparatuses when the new patient information is newly added and stored in a database.

### SUMMARY OF THE INVENTION

In order to achieve the foregoing first object, the invention of claim 1 is a medical information processing system constructed to connect two or more medical information processing apparatuses to one another via the Internet, to convert patient information into e-mail and to transmit the e-mail from one of the two or more medical information processing apparatuses to the other medical information processing apparatuses via the Internet, wherein the medical information processing apparatus on a transmission side includes data recording/reproducing means on the transmission side, data inputting means on the transmission side and data processing means on the transmission side, the data recording/reproducing means on the transmission side has a database for recording patient information, the data inputting means on the transmission side is provided to be able to input the patient information to the database of the data recording/reproducing means on the transmission side, and the data processing means on the transmission side is set to check the database based on a predetermined setting condition, to automatically prepare e-mail attached with new patient information in the database and a patient ID in the information, and to transmit the e-mail to one or more predetermined destination medical information processing apparatuses.

Moreover, the invention of claim 2 is characterized in that the new information in the database is any one of new data newly added, changed data and partially deleted information.

Furthermore, the invention of claim 3 is characterized in that the setting condition is any one of specified time intervals, a previously specified time and activation by some explicit link.

The invention of claim 4 is characterized in that the setting condition is a set capacity of transmittable data as the e-mail.

The invention of claim 5 is characterized in that the set capacity can be set based on a mailbox having a smaller capacity between mailboxes on the transmission side and the receiving side.

The invention of claim 6 according to claim 1 is characterized in that the data processing means on the transmission side is set to prepare a transmission message file to the effect that the e-mail has been transmitted when automatically preparing the e-mail, and to transmit the transmission message file, via a transmission route different from the transmission route of the e-mail, to the one or more predetermined destination medical information processing apparatuses or to facsimiles placed in places where the medical information processing apparatuses are located.

The invention of claim 7 is characterized in that the data processing means on the transmission side is set to prepare a list of the transmission message files and to transmit the list via a transmission route different from the transmission route of the e-mail to any of the one or more predetermined destination medical information processing apparatuses or to the facsimiles placed in the places where the medical information processing apparatuses are located at any of certain specified time intervals and a previously specified time.

The invention of claim 8 according to claim 1 is characterized in that the data processing means on the transmission side is set to prepare a list of the transmission message files and to transmit the list to the one or more predetermined destination medical information processing apparatuses via the same transmission route as the transmission route of the e-mail at any of certain specified time intervals and a previously specified time.

The invention of claim 9 according to claim 8 is characterized in that the medical information processing apparatus on the destination side is set to transmit reply mail to the effect that the list of the transmission message files has been received when receiving the list.

The invention of claim 10 is characterized to serve as a recording medium for an information processing system, the recording medium comprising programs for the medical information processing systems of claims 1 to 7 stored therein.

### Operation

According to the invention of claim 1 as described above, the data processing means of the transmission side checks the database based on the predetermined setting condition, automatically prepares the e-mail attached with the new patient information in the database and the patient ID in the information, and transmits the e-mail to the one or more predetermined destination medical information processing apparatuses. Thus, when there is new information in the database, this new information and the patient ID of the information can be automatically converted into e-mail, and the e-mail can be automatically transmitted to the destination medical information processing apparatuses.

Hence, in the case where the image data, the accompanying patient data and the like are to be shared or copied among a plurality of ophthalmologic image information systems by use of a network such as the Internet connected through a telephone line, an operation, in which an operator him/herself calls out such information from the database and prepares and transmits e-mail having the information attached thereto at each sharing and copying, becomes unnecessary. This prevents occurrence of the problems such as a mistake in designating the destination or the information to be attached to the e-mail, enables necessary new information to be transmitted to the destinations simply, automatically and securely without an error, and enables update of the information (data) in the database of the destinations. In addition, when adding graphic information and observation/diagnosis information to the images sent by e-mail and sending back the images to the transmitter, it is not necessary to designate the destination to be replied to by separately referring to an address book and the like based on the patient information of the images at each sending back as has been done heretofore. This simplifies the procedure for the designation, causes no mistake in designating the destination to be replied to, and prevents the destination itself from being mistaken.

Moreover, according to the invention of claim 2, when there is new data, changed data or partially deleted information in the database, such information and the patient ID of the information are automatically converted into e-mail, and the e-mail is automatically transmitted to the destination medical information processing apparatuses.

Specifically, when there is new text data or new image data newly added and stored in the database, the new text data or the new image data can be automatically converted into the e-mail, and the e-mail can be automatically transmitted to the destination medical information processing apparatuses. Also in this case, in the case where the image data, the accompanying patient data and the like are to be shared or copied among a plurality of ophthalmologic image information systems by use of a network such as the Internet connected through a telephone line, an operation, in which an operator him/herself calls out such information from the database and prepares and transmits e-mail having the information attached thereto at each sharing and copying, becomes unnecessary. This prevents occurrence of the problems such as a mistake in the destination or the information to be attached to the e-mail, enables necessary new information to be transmitted to the destination simply, automatically and securely without an error, and enables update of the information (data) in the database of the destination.

Furthermore, according to the invention of claim 3, the database is checked at the specified time intervals or at the previously specified time, and if there is new text data or new image data newly added and stored in the database, e-mail attached with the new text data or the new image data and the accompanying patient data is automatically prepared, and the e-mail is transmitted to the one or more predetermined destination medical information processing apparatuses. As described above, the information (data) in the database of the destination can be updated at the specified time intervals or at the previously specified time.

According to the inventions of claims 4 and 5, occurrence of a situation such that a transmission data capacity becomes too large to be transmitted can be previously prevented.

According to the invention of claim 6, even if trouble occurs in one of the transmission routes, confirmation is made on the destination side (receiving side) that the e-mail attached with the new text data or the new image data and the accompanying patient data has not been delivered thereto, and retransmission of the e-mail can be requested to the transmission side rapidly.

According to the invention of claim 7, even if trouble occurs in one of the transmission routes, confirmation is made on the destination side (receiving side) at every set time or at specified time intervals that the e-mail attached with the new text data or the new image data and the accompanying patient data has not been delivered thereto, and retransmission of the e-mail can be requested to the transmission side rapidly. In this case, even if trouble occurs in both of the transmission routes, since the transmission message file has not been delivered, the occurrence of trouble at least in the route for the transmission message file between the transmission routes can be known.

According to the invention of claim 8, the data processing means on the transmission side prepares a list of the transmission message files and transmits the list to the one or more predetermined destination medical information processing apparatuses via the same transmission route as the transmission route of the e-mail at specified time intervals or at a previously specified time.

Moreover, according to the invention of claim 9, the medical information processing apparatus on the destination side transmits to the transmission side reply mail to the effect that the list of the transmission message files has been received in the case of receiving the list.

As described in claim 10, each program can be provided as a recording medium for an information processing system. According to the invention of claim 10, by use of the program in the recording medium having the above-described system, when there is new information in the database, it is possible to automatically convert the new information and the patient ID of the information into e-mail and automatically transmit the e-mail to the destination medical information processing apparatuses. Moreover, the effects of claims 1 to 7 can also be expected by use of this recording medium.

### BRIEF DESCRIPTION OF THE DRAWINGS

Fig. 1 is a schematic explanatory view of a medical information processing system according to the present invention.
Fig. 2 is a circuit diagram of a host computer of Fig. 1.
Fig. 3 is a circuit diagram of an information processing terminal of Fig. 1.
Fig. 4 is a circuit diagram of a network controller (a network server) of Fig. 1.
Fig. 5 is an explanatory view of a setting screen of mail transmitting and receiving application software for use in the medical information processing system of Fig. 1.
Fig. 6 is an explanatory view of a detail setting screen displayed by manipulating "Set Detail" of Fig. 5.
Fig. 7 is an explanatory view of a patient ID and a sender.
Fig. 8 is a schematic explanatory view showing a communication example in accordance with the medical information processing system of Figs. 1 to 5.
Fig. 9 is a schematic explanatory view showing another communication example in accordance with the medical information processing system of Figs. 1 to 5.
Fig. 10 is a schematic explanatory view showing a second embodiment of the medical information processing system according to the present invention.
Fig. 11 is a control circuit diagram of a medical information processing terminal shown as a part of Fig. 10.
Fig. 12 is a schematic view for communication in the medical information processing terminal of Fig. 10.
Fig. 13 is a schematic view for determination and communication in the medical information processing terminal of Fig. 10.
Fig. 14 is a schematic view showing one example for determination and communication in the medical information processing system of Fig. 10.
Fig. 15 is a schematic view showing another example for determination and communication in the medical information processing system of Fig. 10.
Fig. 16 is an explanatory view showing a display screen for grading images, shown by means of grading image displaying software in Fig. 13.
Fig. 17 is an explanatory view showing a display example of a grading screen shown by means of grading report preparing software in Fig. 13.

### BEST MODE FOR CARRYING OUT THE INVENTION

Hereinafter, description will be made for embodiments of the present invention with reference to the drawings.

### <First Embodiment>

### [Constitution of First Embodiment]

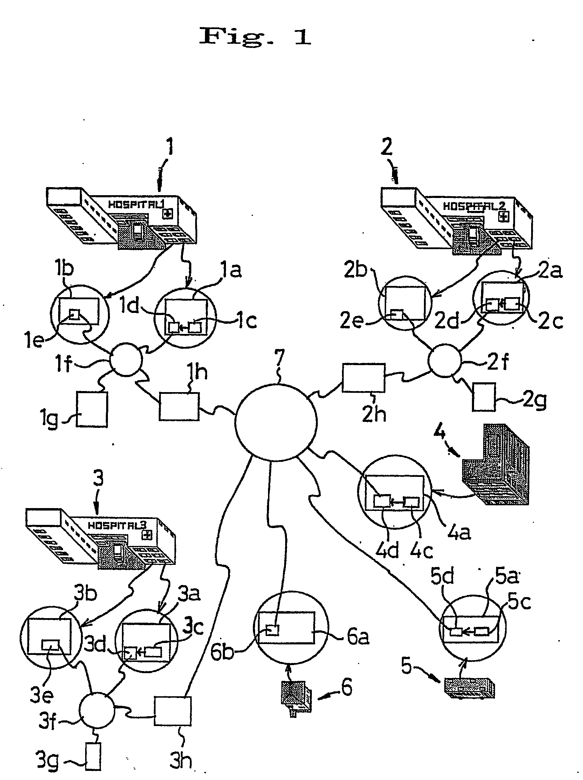

Fig. 1 shows a first embodiment of the present invention.

In Fig. 1, reference numeral 1 denotes a university hospital (hereinafter, referred to as "hospital 1"); numeral 1a denotes an ophthalmologic inspection room of the hospital 1; numeral 1b denotes an ophthalmologic examination room next to the ophthalmologic inspection room 1a; numerals 2 and 3 denote other hospitals (a hospital 2 and a hospital 3); numerals 2a and 3a denote ophthalmologic inspection rooms of the hospitals 2 and 3, respectively; numerals 2b and 3b denote ophthalmologic examination rooms next to the ophthalmologic inspection rooms 2a and 3a, respectively; a numeral 4 denotes a clinic; a numeral 4a denotes an ophthalmologic examination room of the clinic 4; numeral 5 denotes an ophthalmologic examination bus; numeral 5a denotes an ophthalmologic examination room of the ophthalmologic examination bus 5; numeral 6 denotes a house of, for example, an ophthalmologic specialist working at the hospital 1; and numeral 6a denotes a study of the house 6.

In the above-described ophthalmologic inspection rooms (medical inspection rooms) 1a, 2a and 3a, ophthalmologic inspection apparatuses (medical inspection apparatuses) 1c, 2c and 3c, such as slit lamps, tonometers, refractometers, and fundus cameras, are provided as ophthalmologic data (data including ophthalmologic image data) inputting means (medical data inputting means), respectively. Also in the ophthalmologic examination rooms 4a and 5a, ophthalmologic inspection apparatuses 4c and 5c, such as slit lamps, tonometers, refractometers, and fundus cameras, are provided as ophthalmologic data (data including ophthalmologic image data) inputting means (medical data inputting means), respectively.

Moreover, in the ophthalmologic inspection rooms 1a, 2a and 3a, ophthalmologic information processing terminals (ophthalmologic information processing apparatuses) 1d, 2d and 3d for processing inspection data information from the ophthalmologic inspection apparatuses 1c, 2c and 3c are provided as medical information processing terminals (medical information processing apparatuses), respectively. Moreover, in the ophthalmologic examination rooms 4a and 5a, ophthalmologic information processing terminals (ophthalmologic information processing apparatuses) 4d and 5d for processing inspection data information from the ophthalmologic inspection apparatuses 4c and 5c are provided as medical information processing terminals (medical information processing apparatuses), respectively. Furthermore, in the ophthalmologic examination rooms 1b 2b and 3b and the study 6a, ophthalmologic information processing terminals 1e, 2e, 3e and 6b for processing inspection data information are provided as medical information processing terminals (medical information processing apparatuses), respectively. In the case where the ophthalmologic inspection apparatuses 1c to 5c are slit lamps, when the ophthalmologic inspection apparatuses 1c to 5c photograph sectional images of anterior ocular segments by television cameras, the ophthalmologic inspection apparatuses 1c to 5c are set to input right and left distinguishing information of sectional images of photographed eyegrounds and ophthalmologic image data (medical image data, that is, medical image information) to the ophthalmologic information processing terminals 1d to 5d, respectively.

Moreover, in the case where the ophthalmologic inspection apparatuses 1c to 5c are fundus cameras, when the ophthalmologic inspection apparatuses 1c to 5c photograph eyeground images to be inspected by television cameras, the ophthalmologic inspection apparatuses 1c to 5c are set to input patient information such as right and left distinguishing information of photographed eyeground images and eyeground image data (medical image data, that is, medical image information) to the ophthalmologic information processing apparatuses 1d to 5d, respectively. Note that the eyeground images include color images photographed of the eyegrounds, images of eyeground blood vessels by visible fluorescence, images of eyeground blood vessels by infrared fluorescence, eyeground images by infrared rays and the like.

Furthermore, in the case where the ophthalmologic inspection apparatuses 1c to 5c are tonometers, when the ophthalmologic inspection apparatuses 1c to 5c inspect eyes to be inspected for ocular tension data, the ophthalmologic inspection apparatuses 1c to 5c are set to input patient information such as right and left distinguishing information of the inspected eyes and the ocular tension data (medical inspection data, that is, medical inspection information) to the ophthalmologic information processing apparatuses 1d to 5d, respectively.

Then, when the right and left distinguishing information of sectional images of the eyegrounds and the image data (image information) or the ocular tension data are inputted to the ophthalmologic information processing terminals 1d to 5d, the ophthalmologic information processing terminals 1d to 5d record and store the right and left distinguishing information of sectional images of the eyegrounds and the image data (image information) or the ocular tension data in correlation with patient data (patient information) such as patient name, age, sex and patient ID and photographing condition information (photographing condition data) such as photographing date and place.

The ophthalmologic information processing terminals 1d and 1e are connected to a host computer 1g of the hospital 1 and a network controller (network server) 1h via an intranet (internal network) 1f, all of which constitute an internal medical information processing system (internal medical information processing apparatus) A together with medical information processing terminals (not shown) provided in other departments such as internal medicine, surgery and pediatrics and connected to the intranet 1f.

The ophthalmologic information processing terminals 2d and 2e are connected to a host computer 2g of the hospital 2 and a network controller (network server) 2h via an intranet (internal network) 2f, all of which constitute an internal medical information processing system (internal medical information processing apparatus) B together with medical information processing terminals (not shown) provided in other departments such as internal medicine, surgery and pediatrics and connected to the intranet 2f.

The ophthalmologic information processing terminals 3d and 3e are connected to a host computer 3g of the hospital 3 and a network controller (network server) 3h via an intranet (internal network) 3f, all of which constitute an internal medical information processing system (internal medical information processing apparatus) C together with medical information processing terminals (not shown) provided in other departments such as internal medicine, surgery and pediatrics and connected to the intranet 3f.

Moreover, the ophthalmologic information processing terminals 1d to 3d are connected one another via the intranets 1f to 3f, the network controllers 1h to 3h and the Internet (external network) 7, and are connected to the ophthalmologic information processing terminals 4d and 6b via the intranets 1f to 3f, the network controllers 1h to 3h and the Internet (external network) 7, thus enabling communication thereamong. This communication system among the internal medical information processing systems (internal medical information processing apparatuses) A to C via the Internet constitutes the medical information processing system according to the present invention.

Note that the medical inspection data and the medical image data (patient information) include the ones inputted in other departments such as internal medicine, surgery and pediatrics. For example, in the case where a diabetic patient consults in an internal medicine, non-mydriatic fundus photographing and contrast photographing for eyeground blood vessels are performed therefor, whereby a progress state of diabetes and a degree of a visual field can be diagnosed based on an image of the eyeground and an image of the eyeground blood vessels. Meanwhile, when such an eyeground image and an eyeground blood vessel image are photographed in an ophthalmologic department, whether or not the patient suffers from diabetes can be known from the eyeground image and the eyeground blood vessel image. In this case, the patient can be made to undergo examination of internal medicine. In this case, in the case where the ophthalmologic clinic and the internal medicine clinic are managed by medical practitioners, the system of the present invention is used for mutually exchanging data or for exchanging data with departments required for a general hospital such as university hospital, whereby information exchange between the personal hospital or clinic and the general hospital can be executed rapidly and simply.

Note that, in the case where image data (image information) and data obtained by converting the patient information into text are merged into one piece of data and sent, ophthalmologic image information processing means (medical information processing means) such as IMAGEnet capable of transmitting data having text data combined into image data and of obtaining the image data and the text data by separating the text data from the received image data is used, thus making it possible to secure confidentiality of the personal text data of the patient. Furthermore, when transmitting the data having the text data combined into the image data, a mosaic should be applied thereto, thus making it possible to secure the confidentiality better. Moreover, with regard to the text data of each patient, the text data is encoded through a publicly known encoding program, the encoded text data is combined into the image data, and the data having the text data combined into the image data is transmitted, thus the confidentiality may be secured more sufficiently. In this case, the mosaic may be applied thereto, thus making it possible to secure the confidentiality better. In the manners as described above, security can be sufficiently secured even if a dedicated line is not used.

### <Constitution of Each Host Computer>

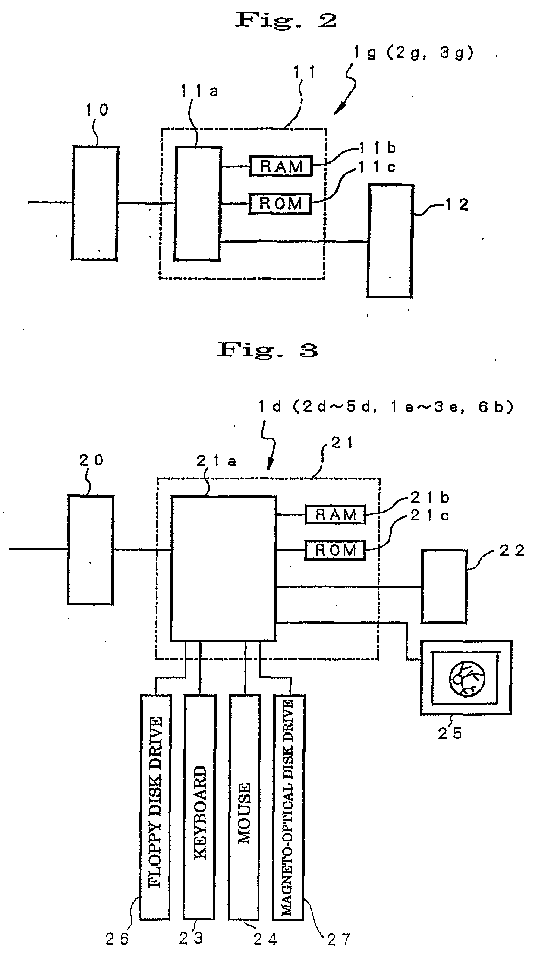

As shown in Fig. 2, each of the host computers (host medical information processing apparatuses) 1g to 3g of the medical information processing systems A to C includes an interface 10 connected to each of the intranets 1f to 3f, a main control circuit 11 as information processing means (data processing means) and other large-capacity data recording/reproducing means (information recording/reproducing means) 12 such as a hard disk and a magneto-optical disk. This main control circuit (main controlling means) 11 includes an operation control circuit (operation controlling means) 11a having a CPU, which is connected to the interface 10, and a RAM 11b and a ROM 11c, which are connected to the operation control circuit 11a Moreover, data recording/reproducing means 12 is connected to the operation control circuit 11a.

Moreover, in the data recording/reproducing means (information recording/reprodncing means) 12, programs for databases such as patient information such as text data (inspection value data) and image data (image information), which are obtained by inspection, and accompanying patient information and photographing condition information (photographing condition data) are recorded and stored. The patient information (information of a patient) includes patient address, name, age, sex, ID, medical history and the like. Moreover, the photographing condition information includes a photographing place or an inspection place, a photographing date or an inspection date, identification of right and left of an inspected eye photographed or inspected, a type of the inspection and the photographing and the like.

### <Constitution of Each Terminal>

Moreover, as shown in Fig. 3, each of the ophthalmologic information processing terminals (ophthalmologic information processing apparatuses) 1d to 5d, 1e to 3e and 6b includes an interface 20 connected to any of the intranets 1f to 3f and the Internet 7, a main control circuit 21 as information processing means (data processing means), and other data recording/reproducing means (information recording/reproducing means) 22 such as a hard disk and a magneto-optical disk. This main control circuit (main controlling means) 21 includes an operation control circuit (operation controlling means) 21a having a CPU, which is connected to the interface 20, and a RAM 21b and a ROM 21c, which are connected to the operation control circuit 21a.

In addition, to the operation control circuit 21a, the data recording/reproducing means 22 is connected. Note that the interface 20 constitutes data inputting/outputting means having a function as data inputting means and a function as data outputting means. Moreover, to the operation control circuit 21a, a keyboard 23, a mouse 24 and the like are connected as data input devices (data inputting means) and a display device (displaying means) 25 such as a CRT (monitor television) and a liquid crystal display device are connected. Note that, in the case where a screen 25a of the display device 25 is of a touch panel system, this touch panel also serves as data inputting means.

Furthermore, to the operation control circuit 21a, a floppy disk drive (data recording/reproducing means) 26, a magneto-optical disk drive (data recording/reproducing means) 27 for a MO, a CD-ROM, a CD-R and the like are connected as data inputting/outputting means.

In the ophthalmologic information processing terminals 1d to 5d, the patient information (information of the patient) such as patient address, name, age, sex, ID and medical history and the photographing condition information (information of the patient) such as a photographing place or an inspection place, a photographing date or an inspection date, identification of right and left of the inspected eye photographed or inspected and a type of the inspection or the photographing are inputted to the operation control circuit 21a by use of the data inputting means such as the keyboard 23, the mouse 24, the touch panel and the like.

Moreover, when the text data (inspection value data) and the image data (image information), which are obtained by the inspection, are inputted, the operation control circuit 21a of each of the ophthalmologic information processing terminals 1d to 5d interrelates, and temporarily records and stores the patient information such as the text data (inspection value data) and the image data (image information), which are obtained by the inspection, accompanying patient information and the photographing condition information (photographing condition data) in the data recording/reproducing means (information recording/reproducing means) 22.

### (Automatic E-mail Preparation/Transmission Function)

Moreover, the ophthalmologic information processing terminal 1d is set to check a database of the data recording/reproducing means (information recording/reproducing means) 12 of the host computer 1g, or to check a database of the data recording/reproducing means 22 of the ophthalmologic information processing terminal 1d via the intranet 1f at specified time intervals or at a previously specified time, then to automatically prepare e-mail having the new patient information such as the new text data and the new image data, accompanying patient data, the photographing condition data, and if necessary, patient information such as old text and image data attached thereto when there is new patient information such as new text data and new image data, which is newly added and stored, in the database, and then to transmit the e-mail to one or more predetermined destination medical information processing apparatuses. Moreover, the ophthalmologic information processing terminal 1d is set to automatically prepare e-mail with the patient information as new patient information and to transmit the e-mail to the one or more predetermined destination medical information processing apparatuses also in the case where a change is added to the patient information or the case where the patient information is partially deleted.

A program of an automatic e-mail preparation/transmission function as described above is recorded and stored in the data recording/reproducing means (information recording/reproducing means) 22 of the ophthalmologic information processing terminal 1d. This program can be previously stored in a recording medium such as a floppy disk and a magneto-optical disk and provided, can be previously recorded and stored in a recording medium such as a hard disk of a specified terminal and provided via a network such as the Internet, or alternatively can be recorded in a recording medium such as a memory and provided.

### (Automatic Received Data Separation/Recording Function)

Moreover, the ophthalmologic information processing terminal 1d is set to separate the new patient information such as new text data and new image data, which is attached to the e-mail, and the accompanying patient data from the e-mail if there is new e-mail received via the intranet 1f at a specified interval or at a previously specified time, and to store the separated information (data) in the database of the data recording/reproducing means 12 of the host computer 1g or the database of the data recording/reproducing means 22 of the ophthalmologic information processing terminal 1d. Furthermore, also in the case of receiving the patient information to which a change is added or the partially deleted patient information, the ophthalmologic information processing terminal 1d is set to separate the patient information as new patient information from the e-mail, and to automatically reflect the separated information on the database of the data recording/reproducing means 12 of the host computer 1g or on the database of the data recording/reproducing means 22 of the ophthalmologic information processing terminal 1d.

A program of an automatic received data separation/recording function as described above is also recorded and stored in the data recording/reproducing means (information recording/reproducing means) 22 of the ophthalmologic information processing terminal 1d. This program can be also stored in the recording medium such as a floppy disk and a magneto-optical disk and provided, can be previously recorded and stored in a recording medium such as a hard disk of a specified terminal and provided via the network such as the Internet, or alternatively can be recorded in a recording medium such as a memory and provided.

Also in the data recording/reproducing means (information recording/reproducing means) 22 of each of the ophthalmologic information processing terminals 2d to 5d, 1e to 3e and 6b, the program having an automatic e-mail preparation/transmission function and an automatic data separation/recording function, which are similar to those in the ophthalmologic information processing terminal 1, are recorded and stored. In the data recording/reproducing means (information recording/reproducing means) 22 of each of the ophthalmologic information processing terminals 4d, 5d, 6b, 2e and 3e, a program having the automatic e-mail preparation function similar to that in the ophthalmologic information processing terminal 1d is recorded and stored.

The program having the automatic e-mail preparation/transmission function and the automatic data separation/recording function, which are similar to those in the ophthalmologic information processing terminal 1d, can be also imparted to the host computers (host medical information processing apparatuses) 1g to 3g and the network controllers 1h to 3h of the medical information processing systems A to C.

Note that the above-described program having the automatic e-mail preparation/transmission and the automatic received data separation/recording functions are incorporated in application software for mail transmission/receiving, and in this application software for mail transmission/receiving, for example, settings described below are possible, including a timing of transmission/receiving.
∗ Setting as to whether only either transmission or receiving is performed or both thereof are performed
∗ Setting as to whether or not transmission/receiving is performed immediately after activation
∗ Setting of a time zone when transmission/receiving is performed (starting/ending time)
∗ Setting of an interval when transmission/receiving is performed (separately set for transmission and receiving)

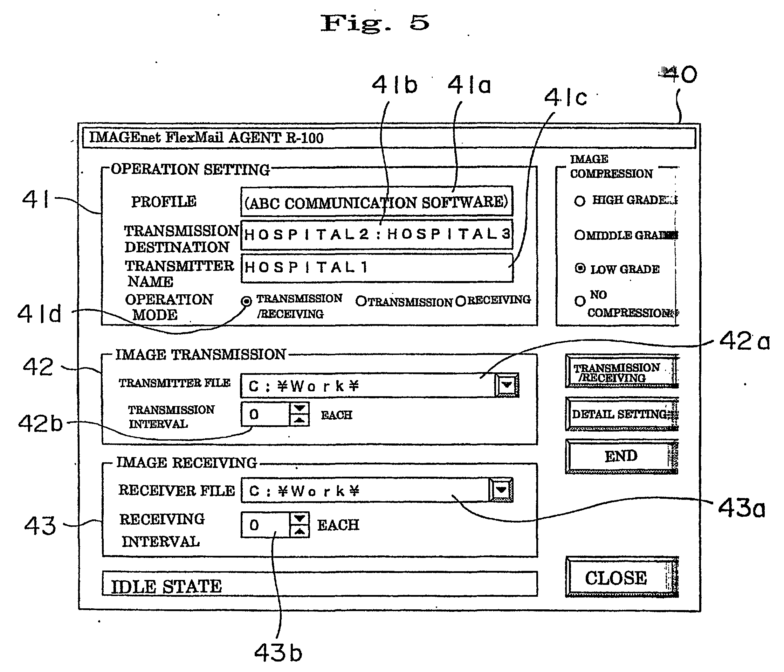

Specifically, as shown in Fig. 5, on a monitor screen 40, there can be displayed an operation setting column 41, an image transmission column 42, an image receiving column 43 and the like, which are brought by the application software for mail transmission/receiving.

On this operation setting column 41, there are provided a profile setting frame 41a for communication software, a setting frame 41b for communication destinations and a setting frame 41c for a transmitter name as well as a setting portion 41d for transmission/receiving, a setting portion 41e for transmission, a setting portion 41f for receiving and the like.

Moreover, on the image transmission column 42, an input frame 42a for inputting a transmitter file (for example, C: Work ¥) and an input frame 42b for inputting a transmission interval are provided. The transmitter file and the transmission interval are set, and one of the setting portion 41d for transmission/receiving and the setting portion 41e for transmission is clicked to put a black circle as a setting mark thereonto, thus making it possible to set the automatic e-mail preparation/transmission function.

Furthermore, on the image receiving column 43, an input frame 43a for inputting a receiver file (for example, C: Work ¥) and an input frame 43b for inputting a receiving interval are provided. The receiver file and the receiving interval are set, and one of the setting portion 41d for transmission/receiving and the setting portion 41f for receiving is clicked to put a black circle as a setting mark thereonto, thus making it possible to set the automatic received data separation/recording function.

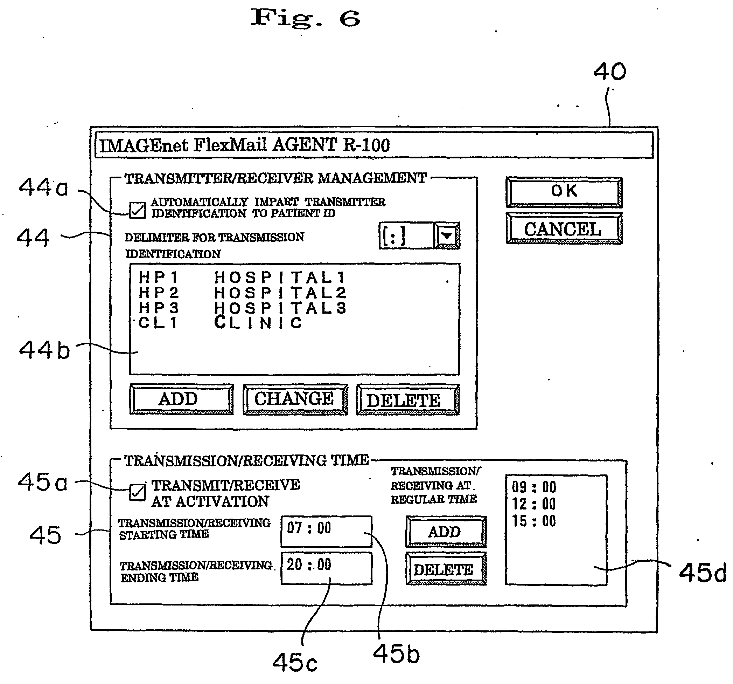

Moreover, a cursor of a mouse (not shown) is set to the "detail setting" displayed on the monitor screen 40 and the mouse is clicked, thus making it possible to make a display as shown in Fig. 6 on the monitor screen 40 by the application software for mail transmission/receiving. In Fig. 6, there are provided a transmitter/receiver management column 44 and a transmission/receiving time column 45, which are brought by the application software for mail transmission/receiving. In this transmitter/receiver management column 44, there are provided a check frame 44a for automatically imparting a transmitter identification to a patient ID, a setting frame 44b for transmitter identification names, and frames for adding, changing and deleting the transmitter identification names. Moreover, in the transmission/receiving time column 45, there are provided a check frame 45a as to whether or not the transmission/receiving is performed at the time of activation, a setting frame 45b for a transmission/receiving starting time, a setting frame 45c for a transmission/receiving ending time, and a setting frame 45d for periodical transmission/receiving time.

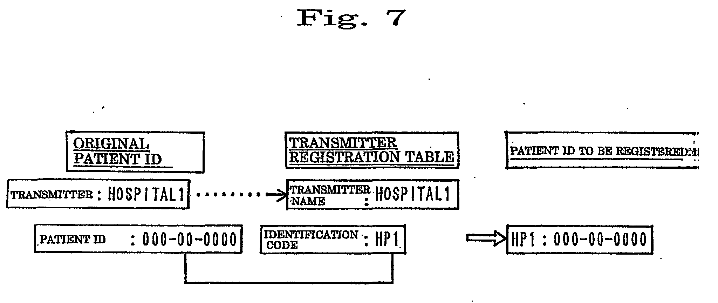

In the case where a check mark is set in the check frame 44a, as shown in Fig. 7, an original patient ID and a patient ID to be registered, which is set by a transmitter registration table, are automatically imparted. Fig. 7 shows the case where the transmitter is the hospital 1, in which a code of 3 orders-2 orders-4 orders, for example, "000-00-0000" is set as a patient ID. Then, in the case where the transmitter name is the hospital 1, if the identification code is set as, for example, HP1, the patient ID to be registered becomes "HP1:000-00-0000".

### <Network Controller>

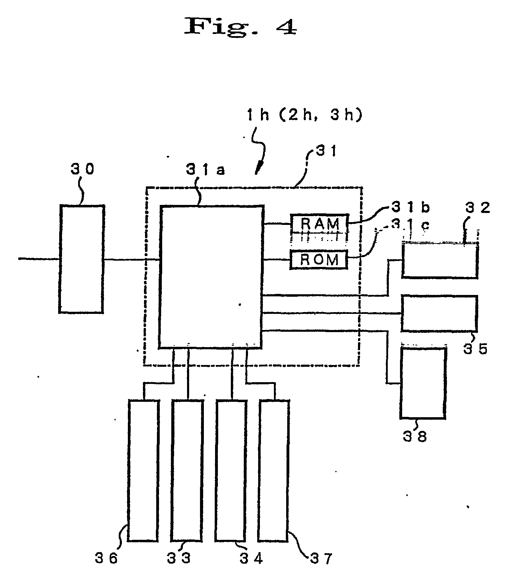

As shown in Fig. 4, each of the network controllers 1h, 2h and 3h includes an interface 30 connected to each of the intranets 1f, 2f and 3f, a main control circuit 31 as information processing means (data processing means) and other data recording/reproducing means (information recording/reproducing means) 32 such as a hard disk and a magneto-optical disk. This main control circuit (main controlling means) 31 includes an operation control circuit (operation controlling means) 31a having a CPU, which is connected to the interface 30, and a RAM 31b and a ROM 31c, which are connected to the operation control circuit 31a.

Moreover, to the operation control circuit 31a, data recoding/reproducing means 32 is connected. Note that the interface 30 constitutes data inputting/outputting means having a function as data inputting means and a function as data outputting means. Moreover, to the operation control circuit 31a, a keyboard 33, a mouse 34 and the like are connected as data inputting means, and a display device (displaying means) 35 such as a CRT (monitor television) and a liquid crystal display device is connected.

Furthermore, to the operation control circuit 31a, a floppy disk drive 36 and a magneto-optical disk drive 37 such as a MO, CD-ROM and a CD-R are connected as data inputting/outputting means. Moreover, in the operation control circuit 31a, an interface 38 connected to the Internet 7 is provided.

In the case where the application software for mail transmission/receiving in which the above-described programs of the automatic e-mail preparation/transmission function and the automatic received data separation/recording function are incorporated is imparted to the network controllers 1h, 2h and 3h, the application software is previously stored in the data recording/reproducing means 32.

### [Specification of Functional Name of Each Processing Apparatus During Transmission or Receiving]

### (Host Computer)

In this embodiment, in the case where each of the host computers (host medical information processing apparatuses) 1g to 3g of the medical information processing systems A to C transmits data (information), the interface 10 as data inputting/outputting means serves as data outputting means on the transmission side, the main control circuit 11 serves as information processing means on the transmission side (data processing means on the transmission side), and the data recording/reproducing means 12 serves as data recording/reproducing means on the transmission side (information recording/reproducing means on the transmission side). Moreover, in the case where each of the host computers (host medical information processing apparatuses) 1g to 3g of the medical information processing systems A to C receives data (information), the interface 10 as data inputting/outputting means serves as data inputting means on the transmission side, the main control circuit 11 serves as information processing means on the receiving side (data processing means on the receiving side), and the data recording/reproducing means 12 serves as data recording/reproducing means on the receiving side (information recording/reproducing means on the receiving side).

### (Terminal)

Moreover, in this embodiment, in the case where each of the ophthalmologic information processing terminals (ophthalmologic information processing apparatuses) 1d to 5d, 1e to 3e and 6b transmits data (information), each data inputting means such as the keyboard 23, the mouse 24 and the touch panel serves as data inputting means on the transmission side. Moreover, in the case where each of the ophthalmologic information processing terminals (ophthalmologic information processing apparatuses) 1d to 5d, 1e to 3e and 6b receives data (information), each data inputting means such as the keyboard 23, the mouse 24 and the touch panel serves as data inputting means on the receiving side. Furthermore, in the case where each of the ophthalmologic information processing terminals (ophthalmologic information processing apparatuses) 1d to 5d, 1e to 3e and 6b transmits data (information), the interface 20 as data inputting/outputting means serves as data outputting means on the transmission side. In the case where each of the ophthalmologic information processing terminals (ophthalmologic information processing apparatuses) 1d to 5d, 1e to 3e and 6b receives data (information), the interface 20 as data inputting/outputting means serves as data inputting means on the receiving side.

Similarly, in the case where each of the ophthalmologic information processing terminals (ophthalmologic information processing apparatuses) 1d to 5d, 1e to 3e and 6b transmits data (information), the operation control circuit 21a of the main control circuit 21 serves as data processing means on the transmission side (information processing means on the transmission side). Moreover, in the case where each of the ophthalmologic information processing terminals (ophthalmologic information processing apparatuses) 1d to 5d, 1e to 3e and 6b receives data (information), the operation control circuit 21a of the main control circuit 21 serves as data processing means on the receiving side (information processing means on the receiving side).

Furthermore, in the case where each of the ophthalmologic information processing terminals (ophthalmologic information processing apparatuses) 1d to 5d, 1e to 3e and 6b transmits data (information), the data recording/reproducing means 22 serves as data recording/reproducing means on the transmission side. Moreover, in the case where each of the ophthalmologic information processing terminals (ophthalmologic information processing apparatuses) 1d to 5d, 1e to 3e and 6b receives data (information), the data recording/reproducing means 22 serves as data recording/reproducing means on the receiving side.

### (Network Controller)

In the case where each of the network controllers (network servers) 1h to 3h transmits data (information), the data inputting means thereof such as the keyboard 33 and the mouse 34 serves as data inputting means on the transmission side. Moreover, in the case where each of the network controllers (network servers) 1h to 3h receives data (information), the data inputting means thereof such as the keyboard 33 and the mouse 34 serves as data inputting means on the receiving side.

Furthermore, in the case where each of the network controllers (network servers) 1h to 3h transmits data (information), the interfaces 30 and 38 as data inputting/outputting means serves as data outputting means on the transmission side. In the case where each of the network controllers (network servers) 1h to 3h receives data (information), the interfaces 30 and 38 as data inputting/outputting means serves as data outputting means on the receiving side.

Similarly, in the case where each of the network controllers (network servers) 1h to 3h transmits data (information), the operation control circuit 31a of the main control circuit 31 serves as data processing means on the transmission side (information processing means on the transmission side). Moreover, in the case where each of the network controllers (network servers) 1h to 3h receives data (information), the operation control circuit 31a of the main control circuit 31 serves as data processing means on the receiving side (information processing means on the receiving side).

Furthermore, in the case where each of the network controllers (network servers) 1h to 3h transmits data (information), the data recording/reproducing means 32 serves as data recording/reproducing means on the transmission side. Moreover, in the case where each of the network controllers (network servers) 1h to 3h receives data (information), the data recording/reproducing means 32 serves as data recording/reproducing means on the receiving side.

### [Operations of First Embodiment]

Hereinafter, description will be made for operations of the above-described system.

For example, description will be made on the premise that an ophthalmologic specialist X (not shown) working at the hospital 1 is periodically sent to the hospitals 2 and 3 and the clinic 4 for examination or examines in the ophthalmologic examination bus 5.

In this case, in the case where the ophthalmologic specialist X stays at the hospital 1, he/she goes to the hospitals 2 and 3 and the clinic 4, he/she examines in the ophthalmologic examination bus 5, or he/she stays at home, it is desirable that he/she can view up-to date inspection data and old inspection data of an ophthalmologic patient anywhere.

Note that, as described above, such inspection data includes, for example, right and left distinguishing information of sectional images of eyegrounds and ophthalmologic image data thereof (ophthalmologic image data (information n)), right and left distinguishing information of eyeground images and eyeground image data thereof (ophthalmologic image data (information)), right and left distinguishing information of inspected eyes and the ocular tension data (inspection data (information)) and the like. The information (data) as described above is recorded together with patient IDs in a patient managing database of the host computer 1g of the hospital 1 or the database of the data recording/reproducing means 22 of the ophthalmologic information processing terminal 1d.

Then, the above-described application software for mail transmission/receiving, in which the program of the automatic e-mail preparation/transmission function and the program of the automatic data separation/recording function are incorporated, is previously recorded in the data recording/reproducing means 22 of the ophthalmologic information processing terminal 1d of the hospital 1. Moreover, the ophthalmologic information processing terminal 1d is always turned on to be operated, and thus the application software for mail transmission/receiving is previously activated.

### (Automatic E-mail Preparation/Transmission)

In this state, the main control circuit (information processing means) 21 of the ophthalmologic information processing terminal 1d of the hospital 1 checks the database of the data recording/reproducing means (information recording/reproducing means) 12 of the host computer 1g via the intranet if at specified time intervals or at specified time. Alternatively, the main control circuit 21 checks the data base of the data recording/reproducing means 22 of the ophthalmologic information processing terminal 1d. If there is new text data or new image data newly added/stored in the database, the main control circuit 21 automatically prepares e-mail attached with the new text data and the new image data, patient data accompanied with those data, photographing condition data, old text data and image data if necessary. Then, the main control circuit 21 transmits the e-mail to one or more of predetermined destination medical information processing apparatuses. Note that the patient information added to the image data also includes graphic information and observation/diagnosis information. Moreover, also in the case where a change is made for the patient information and in the case where the patient information is partially deleted, the main control circuit 21 automatically prepares e-mail with such changed or partially deleted patient information as new patient information, and transmits the e-mail to one or more predetermined destination medical information processing apparatuses.

Moreover, since the ophthalmologic specialist X (not shown) is periodically sent to the hospitals 2 and 3, the clinic 4 and the like for examination and examines in the ophthalmologic examination bus 5, among the ophthalmologic information processing terminals (ophthalmologic information processing apparatuses) 1d to 5d, 1e to 3e and 6b, the present destinations are the ones other than those to which the inspection data and the ophthalmologic images are transmitted to the host computer 1g together with the patient ID.

For example, in the case where inspection data obtained by inspection in the ophthalmologic inspection apparatus 1c or ophthalmologic image data obtained by photographing in the ophthalmologic inspection apparatus 1c is newly inputted to the database of the data recording/reproducing means 22 of the ophthalmologic information processing terminal 1d, or in the case where the inspection data obtained by inspection in the ophthalmologic inspection apparatus 1c or the ophthalmologic image data obtained by photographing in the ophthalmologic inspection apparatus 1c is newly inputted to the database of the data recording/reproducing means (information recording/reproducing means) 12 of the host computer 1g via the ophthalmologic information processing terminal 1d and the intranet 1f, since the inspection data and the ophthalmologic images are recorded in the ophthalmologic information processing terminal 1d, the destinations are the ophthalmologic information processing terminals (ophthalmologic information processing apparatuses) 2d to 5d, 1e to 3e and 6b except for the ophthalmologic information processing terminal (ophthalmologic information processing apparatus) 1d. Moreover, in the case where patient data prepared in the ophthalmologic information processing terminal 1e is newly inputted to the database of the data recording/reproducing means (information recording/reproducing means) 12 of the host computer 1g via the intranet 1f since the patient data is recorded in the ophthalmologic information processing terminal 1e, the destinations are the ophthalmologic information processing terminals (ophthalmologic information processing apparatuses) 1d to 5d, 2e to 3e and 6b.

Thus, the ophthalmologic specialist X can share (view) the information such as the inspection data and the ophthalmologic images transmitted together with the patient ID, in the ophthalmologic examination room 1b next to the ophthalmologic inspection room 1a, the ophthalmologic inspection rooms 2a and 3a of the other hospitals 2 and 3, the ophthalmologic examination rooms 2b and 3b next to the ophthalmologic inspection rooms 2a and 3a, the ophthalmologic examination room 4a of the clinic 4, the ophthalmologic examination room 5a of the ophthalmologic examination bus 5 and the study 6a of the house 6 of the ophthalmologic specialist X working at the hospital 1,

### (Automatic Received Data Separation/Recording)

Meanwhile, if there is any new e-mail received via the intranet If at specified time intervals or at specified time, the main control circuit (information processing means) 21 of the ophthalmologic information processing terminal 1d separates new text or image data and accompanying patient data, which are attached to the e-mail, from the e-mail, and automatically stores the separated data in the database of the data recording/reproducing means 12 of the host computer 1g or in the database of the data recording/reproducing means 22 of the ophthalmologic information processing terminal 1d.

Moreover, also in the case where the patient information for which any change is made or the partially deleted patient information is received, the main control circuit (information processing means) 21 of the ophthalmologic information processing terminal 1d separates the patient information from the e-mail as new patient information. Then, the main control circuit 21 automatically stores the separated information in the database of the data recording/reproducing means 12 of the host computer 1g or in the database of the data recording/reproducing means 22 of the ophthalmologic information processing terminal 1d.

### (Other 1)

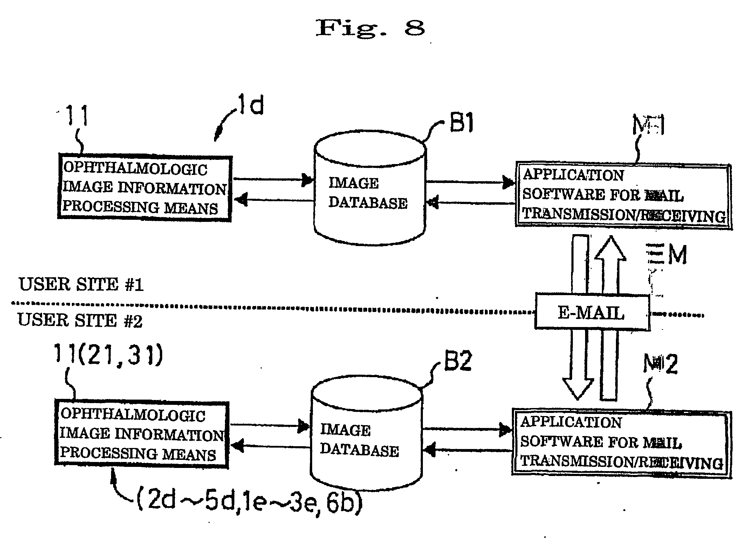

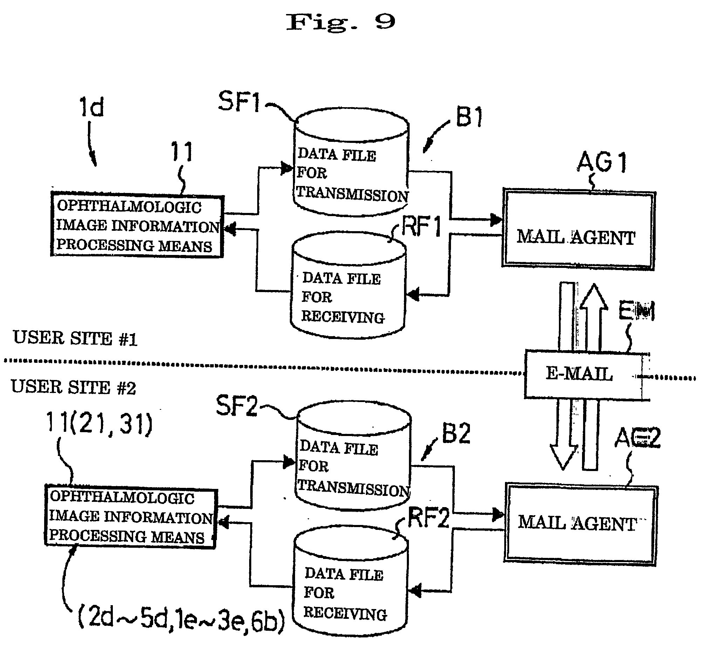

Fig. 8 and Fig. 9 illustrate examples where the ophthalmologic information processing terminal (ophthalmologic information processing apparatus) 1d side becomes a user site #1 which is a transmission side, and where the ophthalmologic information processing terminals (ophthalmologic information processing apparatuses) 2d to 5d, 1e to 3e and 6b side becomes a user site #2 which is a receiving side.

On the user site #1 side in Fig. 8, medical images in an image database B1 are attached to e-mail and transmitted, by use of application software M1 for mail transmission and receiving. Meanwhile, on the user site #2 side, the e-mail attached with the medical images are received by use of application software M2 for mail transmission/receiving, and the medical images are separated from the received e-mail, and then recorded in an image database B2 on the user site #2 side.

Moreover, the database B1 in Fig. 8 includes a data file SF1 for transmission and a data file RF1 for receiving as shown in Fig. 9, and the database B2 in Fig. 8 includes a data file SF2 for transmission and a data file RF2 for receiving as shown in Fig. 9. Note that, in Fig. 9, application software M1 and M2 for mail transmission/receiving in Fig. 8 adopt agents AG1 and AG2 styles which are resident in the CPUs of 11 and 11 (21, 31), respectively.

### (Other 2)

Note that the ophthalmologic information processing terminals 2d to 5d, 1e to 3e and 6b function similarly to the ophthalmologic information processing terminal 1d.

### <Second Embodiment>

### [Constitution]

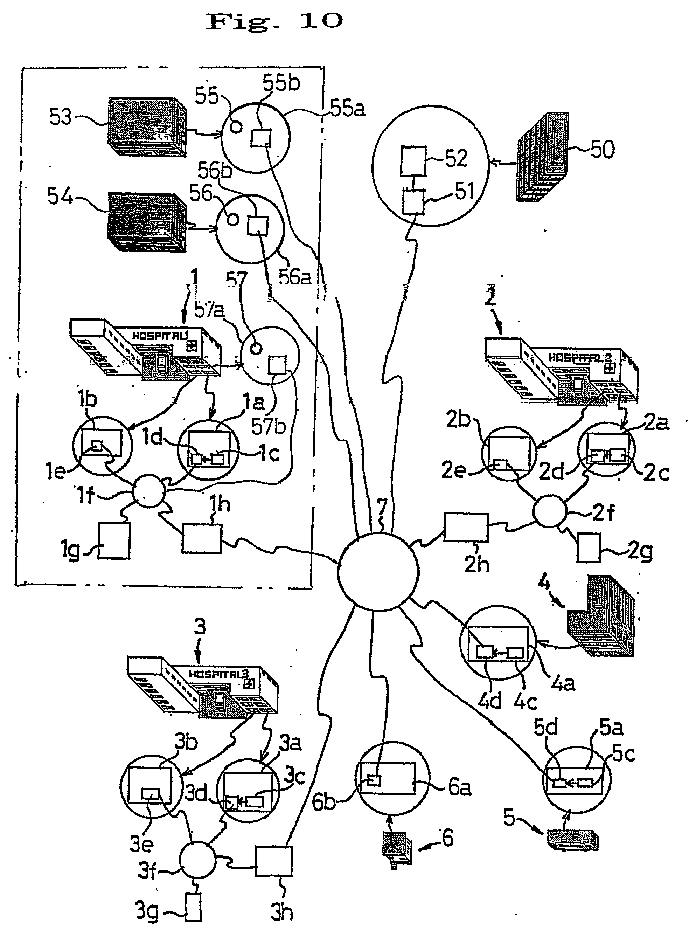

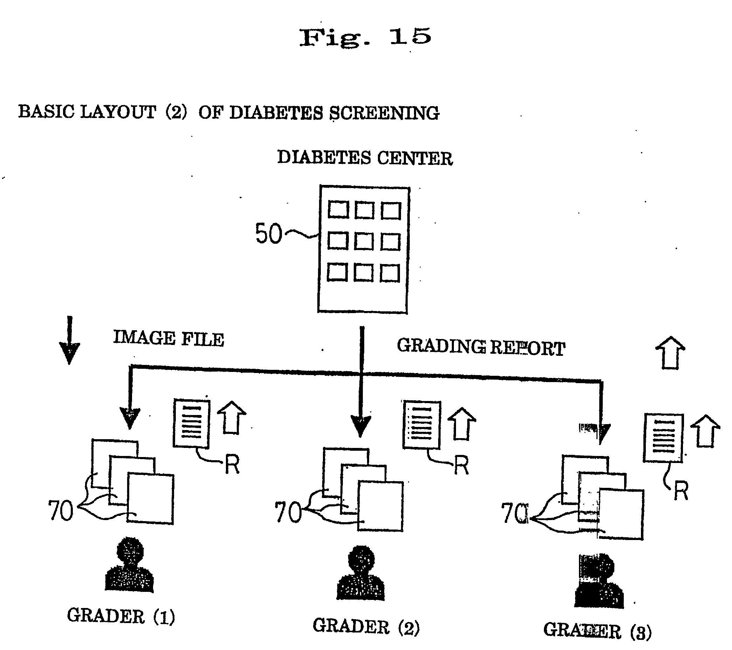

Figs. 10 to 17 show a second embodiment of the present invention. In addition to the constitution, the operation and the effect of the first embodiment, the second embodiment concretely shows a system for screening of a specific disease. In this embodiment, a system for screening of diabetes is shown. Note that the system of this embodiment can be used for screening of diseases other than diabetes. Hereinafter, description will be made for the second embodiment with the system for screening of diabetes as an example.

In Fig. 10, in addition to the constitution of Fig. 1, a center for screening a specific disease, for example, a diabetes center 50 is provided. In this diabetes center 50, a host computer (center-side medical information processing apparatus) 51 is provided as a host computer for specific disease screening. To this host computer 51, an ophthalmologic information processing terminal (ophthalmologic information processing apparatus) 52, which is a medical information processing terminal (center-side medical information processing apparatus), is connected as a specific disease information processing terminal (specific disease information processing apparatus) for screening the specific disease. The host computer 51 is connected to the Internet 7.

Moreover, in the host computer 51, for example, addresses, names, telephone numbers, facsimile numbers and mail addresses of hospitals 53 and 54 managed by specialists (physician or ophthalmologist) 55 and 56 for grading diabetes from an ophthalmologic disease are registered. Furthermore, in the host computer 51, for example, an address, a name, a telephone number, a facsimile number, a mail address of a hospital where a specialist (physician or ophthalmologist) 57 for grading diabetes from an ophthalmologic disease, and a name and a mail address of the specialist are registered. In internal medical examination rooms 55a, 56a and 57a where the specialists 55, 56 and 57 work, ophthalmologic information processing terminals (ophthalmologic information processing apparatuses) 55b, 56b and 57b, which are medical information processing terminals (specialist-side medical information processing apparatuses), are disposed as specific disease information processing terminals (specific disease information processing apparatuses) for specific disease screening. The ophthalmologic information processing terminals 55b and 56b are connected to the Internet 7, and the ophthalmologic information processing terminal 57b is connected to the Internet 7 via the intranet 1f and the network controller 1h.

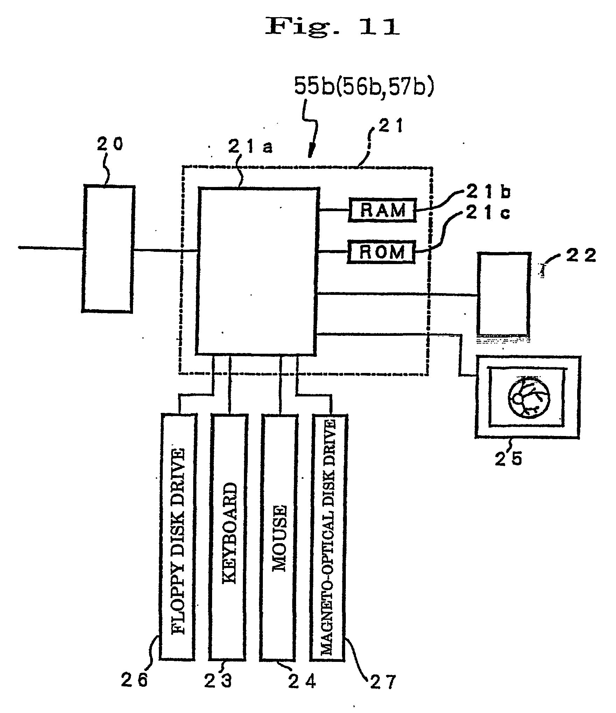

As shown in Fig. 11, each of the ophthalmologic information processing terminals (ophthalmologic information processing apparatuses) 55b, 56b and 57b adopts approximately the same system as each of the ophthalmologic information processing terminals (ophthalmologic information processing apparatuses) 1d, 2d to 5d, 1e to 3e and 6b.

Furthermore, to each of the ophthalmologic information processing terminals 1d, 2d to 5d, 1e to 3e, 6b, 55b, 56b and 57b and the host computers 1g, 2g and 3g, a diabetes screening system having files constituted as below and programs is connected in addition to the constitution of the above-described first embodiment.

### (1) Diabetes Screening System

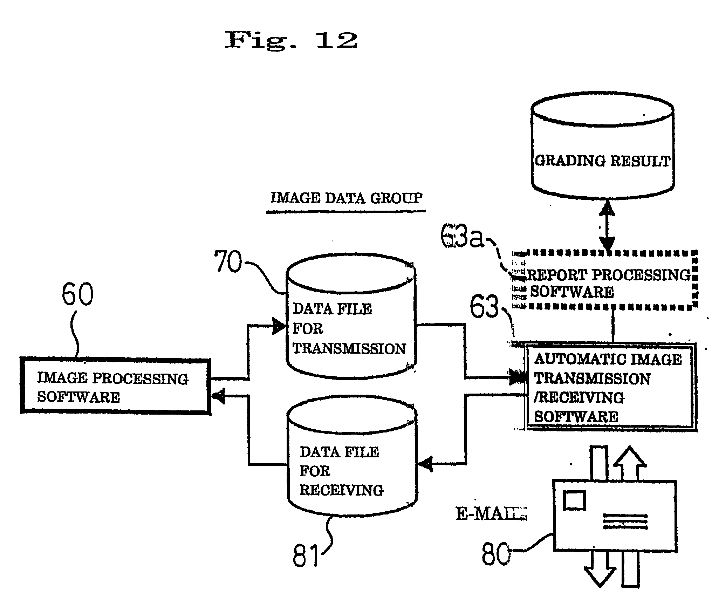

This diabetes screening system is constituted by software components to be described below under a wide-range telescreening environment based on data transfer via the Internet. Each software of this diabetes screening system is installed and recorded in the data recording/reproducing means (information recording/reproducing means) 12, 22 and the like of the ophthalmologic information processing terminals 1d, 2d to 5d, 1e to 3e, 6b, 55b, 56b and 57b and the host computers 1g, 2g and 3g (refer to Figs. 12 to 15).
∗ Image processing software (image processing means) 60
∗ Grading image display software (grading image displaying means) 61
∗ Grading report preparation software (grading report preparation means) 62
∗ Automatic image transmission/receiving software (automatic information transmitting/receiving means) 63
∗ Automatic data transmission/receiving software (automatic information transmitting/receiving means) 64

Each of the components has the following functions (a) to (e), respectively.

### (a) Image processing software 60

The program of the image processing software 60 is used for the original purpose of photographing, storing and managing eyeground images. Moreover, the image processing software 60 is used also as a platform of the grading image display software 61 used by a grader.

This image processing software 60 is used for calling out the grading image display software 61, the grading report preparation software 62 and the like, thus allowing the grading image display software 61 to display grading images and allowing the grading report preparation software 62 to display images for which a grading report is prepared.

### (b) Grading image display software 61

This grading image display software 61 provides image display functions (for example, rapid image display switching or rearranging) suitable for grading. Moreover, the grading image display software 61 is constituted so that this software 61 is capable of calling out the grading report preparation software 62 during display of images to be graded, and capable of readily performing grading, and storing and transmitting the grading result extremely easily.

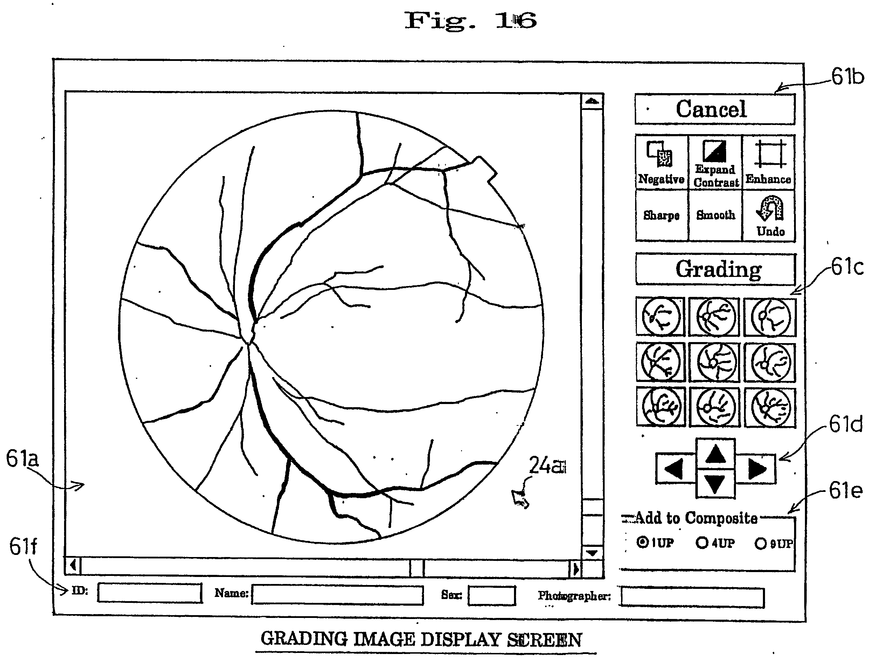

The grading image display software 61 is called out by the image display mechanism of the image processing software 60 and allows the software 60 to display grading images as shown in Fig. 16. As shown in Fig. 16, a screen of the grading image display software 61 called out by the image display mechanism of the image processing software 60 includes a display portion 61a for an eyeground image (medical image), a manipulation menu display portion 61b showing "Cancel", "Negative", "Expand Contrast", "Enhance", "sharp", "Smooth", "Undo" and "Grading", an image display portion 61c for a plurality of eyeground images (nine images in the drawing) for grading, a cursor key display portion 61d used for image selection and others and a display portion 61e for adding images to a composite. Moreover, the screen of the grading image display software 61 includes a patient information display portion 61f for "ID", "Name" and "Sex" and a display portion 61g for "Photographer".

### (c) Grading report preparation software 62

The grading report preparation software 62 is for supporting actual grading, which is prepared by each supplier in accordance with specifications required in each region concerned. Usually, this software has functions of printing a grading result in an original grading form, storing the result in a database file, or transmitting the result via the Internet or the like.

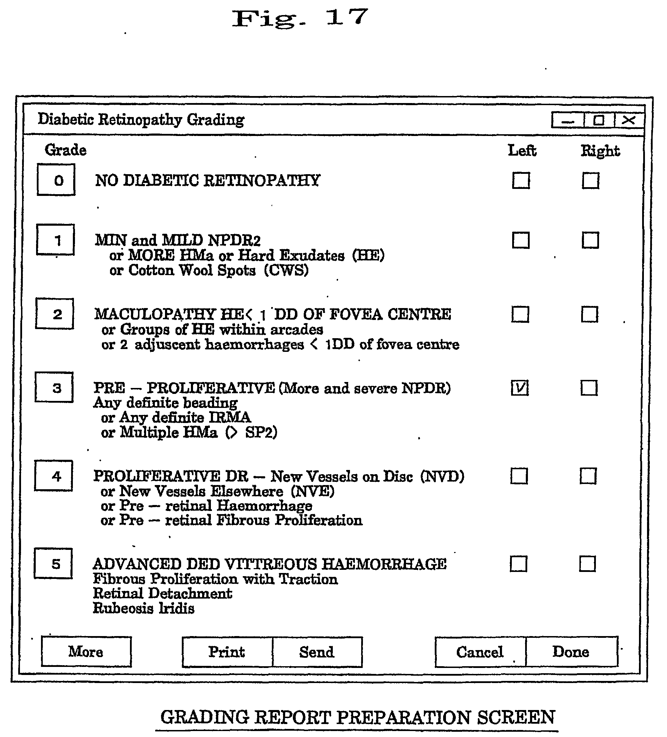

This grading report preparation software 62 is called out by the image display mechanism of the image processing software 60 and allows an image for grading report preparation as shown in Fig. 17 to be displayed. A screen of the grading report preparation software 62 called out by the image display mechanism of the image processing software 60 includes items 62a showing grades for a diabetes grading report, which are denoted by "0" to "5" in Fig. 17, check boxes 62b for a left eye, each of which is provided at the right of each item, check boxes 62c for a right eye, each of which is provided at the right of each item, a menu display portion 62d showing "More", "Print", "Send", "Cancel" and "Done", and the like for operation.

The grading report preparation software 62 is the one which is prepared optimally by each supplier depending on the actual situation of a use region, and specifications unique to a region or a user, such as grading parameters and a printing output form, can be added to the software.

The grading result prepared by the grading report preparation software 62 is stored in the database file (grading result file). Each record in this database file includes at least information as described below.
∗ Patient ID
∗ Grader ID, grader name and grader' department
∗ Time and date of grading
∗ Grading result (contents of which change depending on each medical system such as ophthalmologic, medical and surgical systems)
∗ Time and date of transmission

In this database file, two or more records concerning the same patient, which are prepared by different graders, are stored in some cases.

### (d) Automatic image transmission/receiving software (automatic information transmitting/receiving means) 63

The automatic image transmission/receiving software (automatic image transmission/receiving program) 63 is an automatic transmission/receiving program for an image, which is originally prepared for the purpose of sharing an image of the data file (database) among a plurality of sites. This automatic image transmission/receiving software (automatic image transmission/receiving means) 63 is combined with a report processing software (extension DLL) 63a that can be used for processing data (information) such as a grading report, thus making it possible to automatically transmit both of the images used for grading and the grading result to any destination via the Internet. In this embodiment, such combination enables transmission/receiving of both of the images used for grading and the grading result.

Moreover, also on the receiving side, the automatic image transmission/receiving software (automatic image transmission/receiving program) 63 is used, thus making it possible to automatically store the received images and the grading result in a database of its own.

The automatic image transmission/receiving software (automatic image transmission/receiving program) 63 itself is a standard component; however, for transmission/receiving of the screening grading result, it becomes necessary to add and provide a function extension DLL thereto in accordance with a specification requested by each user.

### (e) Automatic data transmission/receiving software (automatic information transmitting/receiving means) 64

The automatic data transmission/receiving software (automatic data/record transmission/receiving program) 64 is a simple database/file synchronization program, the general usability of which has been enhanced by leaving only the basic functions, such as transmission/receiving of e-mail, of the automatic image transmission/receiving software (automatic image transmission/receiving means) 63 therein and providing system-dependent functions such as access to databases/files as DLL to the outside.

An individual database/file access DLL suitable for each screening system is provided, thus making it possible to automatically transmit only the grading result in the individual database/file to any destination via the Internet. Also on the receiving side, similarly to the above, the automatic data/record transmission/receiving program is used, and thus the grading result can be automatically stored in the database/file of its own.

The automatic data transmission/receiving software (automatic data/record transmission/receiving program) 64 itself is a standard component; however, for transmission/receiving of the screening grading result, it becomes necessary to add and provide thereto software (function extension DLL) for incorporating the specifications required by each user. Such software is added according to needs.

### (2) Supplement

This diabetes screening system is a system having the image processing software 60 as a core thereof. With such a system, the maximum wide ranging of screening can be realized at the minimum cost.

Among the above, only the image processing software 60 and the grading image display software (screening software) 61 are standard components, and other pieces of the software than those are components assumed to require some customization.

### <Operation of Second Embodiment>

Next, description will be made for operations of the medical image processing system of the second embodiment, which is constituted in such a manner as described above. The constitution of the second embodiment also has the operations and the effects of the first embodiment; however, description thereof will be omitted.

As described above, each software of the diabetes screening system is installed and recorded in each of the data recording/reproducing means (information recording/reproducing means) 12, 22 and the like such as the ophthalmologic information processing terminals 1d, 2d to 5d, 1e to 3e, 6b, 55b, 56b and 57b and the host computers 1g, 2g and 3g. Then, each software of diabetes screening system is automatically read in the RAMs 21b and 11b at the time of activating the apparatus.

Note that each software of diabetes screening system may be previously stored in each of the ROM 11c and the ROM 21c and the like, and at the time of activating the apparatus, the software may be automatically read in the RAMs 21b and 11b to be resident therein.

The fundus photography itself is performed not only in the diabetes center 50 but also in ophthalmologic hospitals on the periphery thereof. For example, the fundus photography is performed in the hospitals 1, 2 and 3, the clinic 4, the ophthalmologic examination bus 5, the hospitals 53 and 54, other ophthalmologist's offices and the like. Note that the fundus photography is performed by use of an unillustrated ophthalmologic inspection apparatus (fundus camera) in the diabetes center 50, the ophthalmologic inspection apparatuses 1c 2c and 3c of the hospitals 1, 2 and 3, the ophthalmologic inspection apparatus 4c of the clinic 4, the ophthalmologic inspection apparatus 5c of the ophthalmologic examination bus 5, unillustrated ophthalmologic inspection apparatuses of the hospitals 53 and 54, and ophthalmologic inspection apparatuses in other ophthalmologist's offices. In the case of performing the fundus photography, the ophthalmologic inspection apparatuses 1c to 5c and the other ophthalmologic inspection apparatuses are fundus cameras.

The eyeground images photographed by the ophthalmologic inspection apparatuses 1c to 3c as fundus cameras of the hospitals 1, 2 and 3 are transmitted to the diabetes center 50 via the network controllers 1h, 2h and 3h and the Internet 7. Moreover, the eyeground images photographed by the ophthalmologic inspection apparatus 4c of the clinic 4 and the ophthalmologic inspection apparatus 5c of the ophthalmologic examination bus 5 are transmitted to the diabetes center 50 via the ophthalmologic information processing terminals 4d and 5d and the Internet 7. Furthermore, the eyeground images photographed by the unillustrated ophthalmologic inspection apparatuses of the hospitals 53 and 54 and ophthalmologic inspection apparatuses in other ophthalmologist's offices are also transmitted to the diabetes center 50 via the ophthalmologic information processing terminals 55b and 56b and the Internet 7.

Note that a mechanism for transmission/receiving of an image in this case is not proper to the screening, therefore, a usual mechanism for transmission of an image by e-mail using the Internet can be applied as it is. However, the above-described diabetes screening system can be used.

Specifically, in the hospitals 1, 2 and 3, the clinic 4 and the like, the medical information such as photographed medical images and inspection data are recorded together with the patient information in the recording means (recording apparatus) such as the data recording/reproducing means 22 and the drives 26 and 27 of each of the medical information processing terminals 1d, d, 3d and 4d. Alternatively, the medical information such as photographed medical images and inspection data are recorded together with the patient information in each of the host computers 1h, 2h, 3h and 4h. Moreover, in the ophthalmologic examination bus 5 and the hospitals 53, 54 and 1, for example, the medical information such as photographed medical images and inspection data are recorded together with the patient information in each of the data recording/reproducing means 22 and the drives 26 and 27 of the ophthalmologic information processing terminals (medical information processing terminals) 55b, 56b and 57b. In this case, as such medical information and patient information, for example, the following are enumerated.
(i) Medical information
   * Ophthalmologic information
      ** (Ophthalmologic image)
         Eyeground image
         Cell image of endothelium camerae anterioris
         Image of anterior ocular segment by slit lamp
      ** Inspection data by campimeter
   * Others
      Roentgen image
      CT image
      Image such as MRI image
(ii) Patient information (specific information)
   * Patient ID (ID in hospital)
      <Personal information (individual information)>
   * Name
   * Address
   * Age
   * Sex
   * Medical history

In each of the medical information processing terminals 1d to 4d, the host computers 1h to 4h, the ophthalmologic information processing terminals 55b to 57b, and the like, the image processing software 60, by means of the grading image display software 61, deletes the patient information other than the patient ID, and prepares a data file 70 for transmission, which includes only the patient ID and the medical information. In the hospitals 1, 2 and 3, the clinic 4 and the like, this data file 70 for transmission is recorded in the recording means (recording apparatus) such as the data recording/reproducing means 22 and the drives 26 and 27, alternatively in the host computers 1h to 4h. Moreover, in the ophthalmologic examination bus 5, the hospitals 53, 54 and 1, the data file 70 for transmission is recorded in the recording means (recording apparatus) such as the data recording/reproducing means 22 and the drives 26 and 27 of the ophthalmologic information processing terminals (medical information processing terminals) 55b to 57b.

In this case, the image processing software 60, by means of the grading image display software 61, deletes the patient information other than the patient ID and prepares the data file 70 for transmission, which includes only the patient ID and the medical information. Note that, in the case of the eyeground image as shown in Fig. 16, patient information made of text data such as a patient ID of the eyeground image, a right and left distinguishing indication of the eyeground image and the like is included in the data file 70 for transmission. The patient ID may be a patient ID in a hospital or an ID substituted for the patient ID by a table of random numbers.

Thereafter, in the information processing terminals 1d to 5d and 55b to 57b, the automatic information transmission/receiving software 63 is activated by manipulation of the image processing software 60, and e-mail including (or attached with) the data file 70 for transmission is transmitted via the Internet 7 to the host computer 51 of a medical center for a specific disease, that is, the diabetes center 50 in this embodiment, by use of the automatic information transmission/receiving software 63. This e-mail is received by the host computer 51 of the diabetes center 50 by use of the automatic information transmission/receiving software 63, and the data file 70 for transmission attached to the e-mail is recorded in the host computer 51. Moreover, the data file 70 for transmission, which is prepared in the ophthalmologic image processing terminal 52 of the diabetes center 50, is also recorded in the host computer 51.
126 Incidentally, with regard to concrete methods, systems, criteria and the like for diabetes screening, it is not rare that not only these methods, systems, criteria and the like for diabetes screening differ greatly depending on each country but also each customer's own original specifications exist. A basis of diabetes screening system is to provide the maximum degree of freedom to customers under such a condition while maintaining the portion of the image processing software 60 as a base of the system, by use of the above-described grading image display software 61, grading report preparation software 62, automatic image transmission/receiving software 63 (automatic image transmission/receiving program and individual function extension DLL), automatic data transmission/receiving software (automatic data/record transmission/receiving program and individual function extension DLL) 64.

Now, systemization has been made with the following style as a base. Specifically, in the style, the diabetes center as a center of each region exists, and a plurality of graders for grading images exist on the periphery thereof with regard to diabetes screening. In this case, two types of basic layouts are conceived for information transmission between the diabetes center and the graders. Basic layout 1

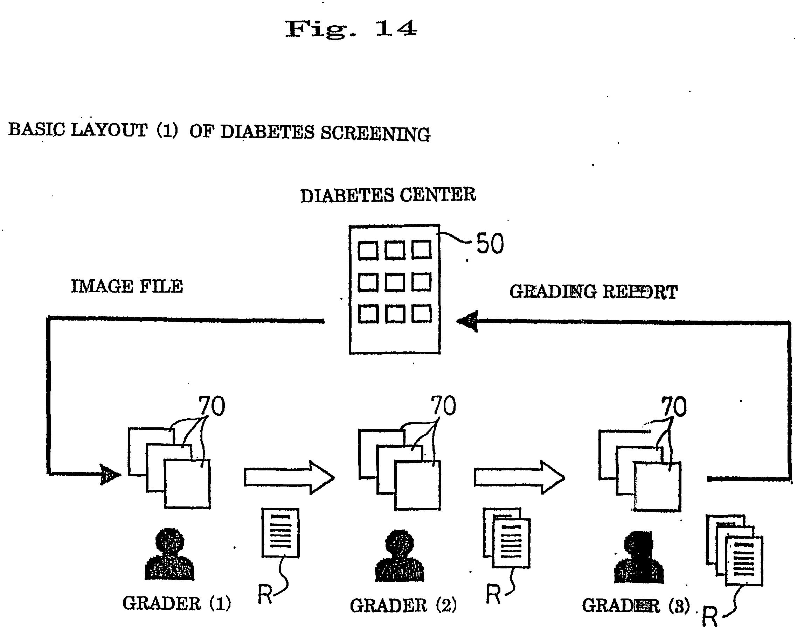

As shown in Fig. 14, one basic layout takes a form, in which the diabetes center 50 sends images to be graded to a certain grader (1), the grader (1) grades the images and then sends the images to the next grader (2) together with a grading result thereof, the grader (2) grades the images and then send the images to the next grader (3) together with a grading result thereof, and the final grader (3) grades the images and then sends grading results of all the graders to the diabetes center 50. Note that, in this case, a system in which each grader cannot refer to the grading results of the other graders is adopted.

Here, assumption is made that the grader (1) is the specialist (physician) 55 of the hospital 53, the grader (2) is the specialist (physician) 56 of the hospital 54, and the grader (3) is the specialist (physician) 57 of the hospital 1.

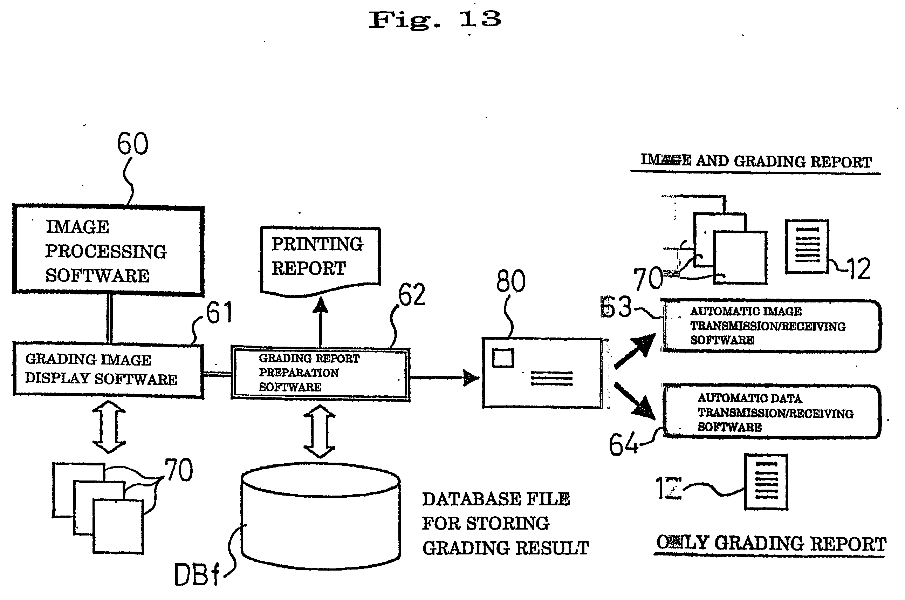

In the ophthalmologic information processing terminal 52 or the host computer 51 of the diabetes center 51, there are installed a medical information processing system including the image processing software 60, the data file 70 for transmission, a data file 81 for receiving, the automatic information transmission/receiving software 63 and the function extension DLL (report processing software) 63a that can be used for transmission of only the grading result, which are shown in Fig. 12. Note that the grading image display software 61 and the grading report preparation software 62 shown in Fig. 13 are also installed in the ophthalmologic information processing terminal 52 or the host computer 51.

Moreover, the medical information processing system similar to that of the diabetes center 50 or the medical information processing system shown in Fig. 13 is installed in each of the ophthalmologic information processing terminals 55b, 56b and 57b of the hospitals 55, 56 and 57.

Among the diabetes center 50, the specialist (physician) 55 of the hospital 53 as the grader (1), the specialist (physician) 56 of the hospital 54 as the grader (2) and the specialist (physician) 57 of the hospital 1 as the grader (3), information is transmitted in a manner to be described below.

### (Preparation and transmission of data file for transmission in the diabetes center 50)

Specifically, in the diabetes center 50, the data file 70 for transmission, which is sent as described above, or the data file 70 for transmission, which is prepared in the ophthalmologic information processing terminal 52, is recorded in the host computer 51. Note that the data file 70 for transmission, which is prepared in the ophthalmologic information processing terminal 52, may be recorded in the recording means (recording apparatus) such as the data recording/reproducing means 22 and the drives 26 and 27 of the ophthalmologic processing terminal 52. The data file 70 for transmission is, for example, the one as shown in Fig. 12.

In this case, as described above, the data file 70 for transmission is set to include the eyeground images and the patient information made of text data such as the patient ID of the eyeground images and the right and left distinguishing indication of the eyeground images, which are as shown in Fig. 16 by means of the grading image display software 61. The patient ID may be a patient ID in a hospital or an ID substituted for the patient ID by a table of random numbers.

Thereafter, the automatic information transmission/receiving software 63 is activated by manipulating the ophthalmologic information processing terminal 52. Then, e-mail 80 including (or attached with) the data file 70 for transmission is transmitted to the ophthalmologic information processing terminal 55b of the hospital 53 where the specialist 55 (grader (1)) works, via the host computer 51 and the Internet 7 by use of the automatic information transmission/receiving software 63. When the e-mail 80 is received by the ophthalmologic information processing terminal 55b of the hospital 53, the automatic information transmission/receiving software 63 of the ophthalmologic information processing terminal 55b separates the data file 70 for transmission from the e-mail 80 and stores the contents of the separated data file 70 for transmission in the data file 81 for receiving.

### (Disease grading and grading result transmission by grader (1))

The specialist 55 activates the image processing software 60 and displays the contents of the received data file 70 for transmission, that is, the eyeground image stored in the data file 81 for receiving as shown in Fig. 16 by use of the grading image display software 61 in Fig. 13, which is activated by the image processing software 60. Then, the specialist 55, based on the display, determines whether or not a diabetes disease is found on the eyeground images displayed.

Next, the specialist 55 displays a screen as shown in Fig. 17, which is to be displayed by the grading report preparation software 62 in Fig. 13 by means of the image processing software 60. On this screen, displayed are the items 62a showing classifications for diabetes grading report, which are denoted by "0" to "5", the check boxes 62b for a left eye, each corresponding to each item, and the check boxes 62c for a right eye, each corresponding to each item. Hence, the specialist 55 displays check marks on the check boxes 62b and 62c by clicking the check boxes 62b and 62c for left and right eyes of items corresponding to the grading result by a mouse and the like, thus preparing the grading result (grading report). This grading result is made to correspond to the patient information, as text information or numerical information, by manipulation of the ophthalmologic image processing terminal 55b, then stored in a database file (grading result file) DBf. This database file (grading result file) DBf is installed in the data recording/reproducing apparatus 22.
Thereafter, the specialist 55 activates the automatic information transmission/receiving software 63 by manipulation of the ophthalmologic information processing terminal 55b. Then, by use of the automatic information transmission/receiving software 63, the specialist 55 prepares the e-mail 80 including (or attached with) the data file 70 for transmission (which includes the eyeground images and the patient information) as weel as a grading report R in the database file DBf having the grading report (grading result) stored therein. Then, the specialist 55 transmits the e-mail 80 to the ophthalmologic information processing terminal 56b of the hospital 54 where the specialist 56 (grader (2)) stays via the Internet 7.

Each record in the database file is set to include at least information as described below.
* Patient ID
* Grader ID, grader name and grader's department
* Time and date of grading
* Grading result (the contents thereof differ depending on each medical system such as ophthalmologic, medical and surgical systems)
* Transmission date

### (Disease grading and grading result transmission by the grader (2))

The specialist 56 as the grader (2) grades the eyeground images concerning the existence and the degree of diabetes, similarly to the specialist 55 as the grader (1), and prepares a database file having a grading report (grading result) stored therein. Moreover, the specialist 56 activates the automatic information transmission/receiving software 63 by manipulation of the ophthalmologic information processing terminal 52. Then, by use of the automatic information transmission/receiving software 63, the specialist 56 transmits the e-mail including the data file 70 for transmission (which includes the eyeground images and the patient information) as well as a grading report R in the database file DBf having the grading report (grading result) stored therein to the. ophthalmologic information processing terminal 57b of the hospital 1 where the specialist 57 (grader (3)) stays via the Internet 7.

### (Disease grading and grading result transmission by grader (3))

The specialist 57 as the grader (3) grades the eyeground images concerning the existence and the degree of diabetes, similarly to the specialists 55 and 56 as the graders (1) and (2), and prepares a database file having a grading report (grading result) stored therein. Moreover, the specialist 57 activates the automatic information transmission/receiving software 63 or 64 for transmitting only the grading report by manipulation of the ophthalmologic information processing terminal 52. Then, by use of the automatic information transmission/receiving software 63 or 64, the specialist 57 transmits only a grading report R in the database file DBf having the grading report (grading result) stored therein to the host computer 51 of the diabetes center 50 via the Internet 7. Here, in the case where only the grading report R in the database file DBf is transmitted by use of the automatic information transmission/receiving software 63, the function extension DLL for transmitting only the grading result (grading report) is used.

Note that, the same database file is used as the database file in which the specialists 55 to 57 (graders (1) to (3)) are to store the grading results. Every time the database file is transmitted by each of the graders (1) to (3), the grading result for the eyeground images of the same patient ID is additionally recorded in the database file. Finally, only the grading report in the database file having the grading results of all the graders (1) to (3) stored therein is transmitted to the diabetes center 50. This grading report (grading result) is stored in the data file for receiving of the diabetes center 50.

Then, in the diabetes center 50, based on the grading report having the grading results of the graders (1) to (3) stored therein, comprehensive final grading is performed as to whether or not symptoms of diabetes are observed in the eyeground images. Then, the comprehensive final grading result is sent back to the hospital, for example, such as the hospitals 1, 2 and 3, which has transmitted the eyeground images to the diabetes center 50 via the Internet 7. Note that the transmission/receiving of e-mail may be automatically performed every time when the contents of the data file 70 for transmission and the database file DBf are changed or updated, similarly to the first embodiment, alternatively may be performed completely automatically.

### Basic layout 2

The basic layout 2 takes a form, in which the diabetes center 50 simultaneously transmits entirely the same images to be graded to all the plural graders (1) to (3), and each of the graders (1) to (3) sends back each grading result directly to diabetes center 50. Time required for all the graders to finish grading can be shorter since the time for data transmission among the graders (1) to (3) is not necessary.

In this case, at the diabetes center 50, prepared is the e-mail 80 including (or attached with) the data file 70 for transmission (which includes the eyeground images and the patient information). Then, by use of the automatic information transmission/receiving software 63, transmitted is the data file 70 for transmission (which includes the eyeground images and the patient information) by the e-mail 80 to the ophthalmologic information processing terminals 55b, 56b and 57b of all the graders (1) to (3), for example, the specialists 55 to 57, via the Internet 7.

As described above, each of the graders (1) to (3) grades the eyeground images concerning the existence and the degree of diabetes, and individually prepares the database file DBf having the grading report (grading result) stored therein. Then, each of the graders (1) to (3) activates the automatic information transmission/receiving software 64 for transmitting only the grading report by manipulation of the ophthalmologic information processing terminal 52. Then, by use of the automatic information transmission/receiving software 64, each of the graders (1) to (3) individually transmits only the grading report in the database file DBf having the grading report (grading result) stored therein to the host computer 51 of the diabetes center 50 via the Internet 7.

Subsequently, at the diabetes center 50, integrated is the grading reports in the host computer 51 in which the individual grading results of the graders (1) to (3) are stored. Then, performed is comprehensive final grading as to whether or not symptoms of diabetes are observed in the eyeground images. Next, the comprehensive final grading result is sent back to the hospital, for example, the hospitals 1, 2 and 3, which has transmitted the eyeground images to the diabetes center 50, via the Internet 7.

### (Other contents of grading report)

In the case where the symptoms of diabetes are observed in the eyeground images, when each of the graders (1) to (3) designates a portion or a range, where the diabetes symptoms are shown, on the eyeground images by mouse, coordinate information (position information) of the designated portion or range is digitalized and stored in the database file DBf. When preparing the grading report from the database file DBf, the digitalized coordination information (position information) is added to the grading report, and the grading report is transmitted to the diabetes center 50.

In the diabetes center 50, based on the coordinate information (position information) of the portion or the range of diabetes symptoms, which is included in the grading report transmitted thereto, a designated state (dots, a frame, diagonal lines, coloring, shading and the like) of the portion or the range of diabetes symptoms, designated by the mouse 24 and the cursor 24a manipulated to be moved on the medical image by the mouse 24, may be reproduced on the eyeground image.

### (Confirmation of file for transmission)

Moreover, in the medical information processing terminals 1d to 4d, the host computers 1h to 4h and 51, the ophthalmologic information processing terminals 55b to 57b and the like, in the case of transmitting the data file 70 for transmission and the grading report R to a destination via a first contracted provider and the Internet 7 through a transmission route using the automatic information transmission/receiving software 63 or 64, a transmission message file is prepared by the automatic information transmission/receiving software 63 or 64.

Subsequently, the transmission message file is transmitted to the same destination through a transmission route via a second contracted provider and the Internet 7; in this case, the message file is transmitted to the terminal or the facsimile of the same destination via a usual transmission route such as ISDN public network and analogue public network.

With the transmission of the transmission message file, even if the transmitted information such as the data file 70 for transmission and the grading report R is not delivered to the destination, the destination side (receiving side) can confirm that some information has been transmitted thereto, can send back a message to the effect that the transmitted information is not delivered yet to the transmitter, and can ask the transmitter to transmit the information one more time.

If the transmission message files described above are listed on the transmission side or the receiving side, a file list thereof can be transmitted at a regular interval in the case where the file is listed on the transmission side, and the file list can be opened at a regular interval in the case where the file is listed on the receiving side.

By preparing the transmission file list and transmitting the transmission file list to the destination, alternatively by opening the transmitted file list on the receiving side, periodically at a regular interval (for example, at 5:00 p.m. every day), it is possible to confirm whether or not the data file 70 for transmission and the grading report has been securely sent to the destination. This file list is transmitted even when there is no information transmitted, whereby the receiving side can grasp that no information has been transmitted thereto.

Since such a list is transmitted at a fixed time, when the list is not transmitted, it is understood that some trouble occurs in the transmission route of the list, and the trouble can be checked.

Moreover, the data processing means on the transmission side can be set so as to prepare a list of the transmission message files and to transmit the list to one or more predetermined destination medical information processing apparatuses, via the same transmission route as the transmission route of e-mail, at specified time intervals or at a previously specified time. In this case also, by confirming whether or not a transmission message file exists at specified time intervals or at a previously specified time on the receiving side, when the transmission message file list is transmitted via the same route as this information transmission route, even in the case where some trouble occurs in the transmission route and the medical information is delivered to the destination late, it is understood that some trouble occurs in this route. Moreover, by confirming whether or not a transmission message file list exists, at specified time intervals or at a previously designated time on the receiving side, what kind of medical information is transmitted thereto is understood from the transmission message file list. If there is any information that has not been delivered thereto, retransmission thereof can be requested. The same can be said even in the case of other routes.

In this case, when the medical information processing apparatus on the destination side receives the transmission message file list, the medical information processing apparatus transmits replying mail to the transmission side to the effect that the list is received to the transmission side. In this case, the transmission side can confirm that the destination has received the transmission message file list. As a result, when the transmission message file list is transmitted via the same route as this information transmission route, even in the case where some trouble occurs in this route and the medical information is delivered to the destination late, it is understood on the transmission side that the medical information is not delivered to the destination at a scheduled time since there is no replying mail. Thus, some measures can be taken.

The above-described programs for information transmission/receiving in the first and second embodiments can be provided as recording media for information processing systems.

### (Transmission data capacity and transmission timing)

As described above, the medical information transmission/receiving in the second embodiment may be automatically performed every time when the contents of the data file 70 for transmission and the database file DBf are changed or updated similarly to the first embodiment, alternatively may be performed completely automatically. In this case, automatic information transmission/receiving in consideration of conditions to be described below may be performed.

Incidentally, in the case where an ophthalmologic image, for example, a color eyeground image is prepared for transmission, the image has a data capacity of 30 K to 40 K even as compressed data. In the case where such eyeground images are synthesized into a panoramic image while changing a photographing position, for example, in the case where nine eyeground images are photographed while changing a photographing position and the nine eyeground images are synthesized into a panoramic image, the total capacity is nine times as much as the capacity of 30 K to 40 K (approximately 270 to 360 K). In addition, in the case where such a panoramic image is prepared for each of right and left eyes, the total capacity is twice the capacity of 270 to 360 K (that is, 540 to 720 K). Moreover, in fluorescence photographing, assuming that about 36 images, for example, are photographed per one eye, then the number of the total images for the right and left eyes are 72, and the total image capacity for the 72 images is seventy two times as much as the capacity of 30 K to 40 K (that is, 2160 to 2880 K). As described above, even for the ophthalmologic image data for one person, the image capacity thereof becomes extremely large.

As a result, in an ophthalmologic examination and in a large hospital or an ophthalmologic clinic, when ophthalmologic images are photographed for a number of patients in a single day, the total capacity of the ophthalmologic images becomes very large. In the case where such a large-capacity data (medical information) is transmitted at a regular interval, also conceived is the case where the entire data cannot be transmitted completely due to the mailbox capacity limit of the provider.

Hence, the following is recommended to the transmission side. Specifically, the transmission side should always check whether or not the medical information capacity in a medical folder, in which medical information such as ophthalmologic images is stored, reaches a set capacity by means of the automatic information transmission/receiving software 63 and 64. Then, when the medical information, such as ophthalmologic images, in the medical folder reaches the set capacity, the medical information in the medical folder should be automatically transmitted to the designated destinations by means of the automatic information transmission/receiving software 63 and 64 in such a manner as in the first embodiment. Thus, data of a certain capacity or more should not be transmitted to the destinations. The set capacity in this case is allowed to be set by means of the automatic information transmission/receiving software 63 and 64 on the transmission side in accordance with the mailbox capacity of the contracted provider.

Moreover, the transmission side and the receiving side do not always join the same provider. In addition, the mailbox capacity differs depending on the provider. In this case, since the transmittable information capacity is limited to the capacity of a smaller mailbox, the set capacity of the transmittable information should be set to correspond to the capacity of the smaller mailbox of the provider on the transmission side.

Furthermore, information of a capacity more than a receiving capacity of the mailbox cannot be received on the receiving side. Therefore, it is recommended, in the case where the capacity of the medical information attached to e-mail is large, that the transmission side should be allowed to transmit the e-mail including medical information to the receiving side only after the receiving side opens (downloads) the mail transmitted from the mailbox of the contracted provider, and thereby the mail is deleted from the mailbox. In this case, it is also recommended that the receiving side should transmit a message to the transmission side of the medical information to the effect that the mail has been downloaded from the mailbox, and that the automatic information transmission/receiving software 63 on the transmission side of the medical information automatically transmits the next e-mail, including the medical information, to the same destination when receiving the message.

Moreover, it is recommended that the set capacity of the transmitted medical information should be changed depending on whether transmission is via dial-up or continuous connection. In the case of dial-up, even in the case where it takes only about 10 seconds to transmit medical information including one medical image, transmission time allowed by a basic rate is set to a specified time length (for example, 3 minutes). In addition, in this case, the time for connection to the provider is included. Hence, in consideration of a telephone charge for connection, by checking whether or not medical information having a capacity transmittable within a specified time (3 minutes) is accumulated in the medical folder, and by transmitting the medical information in the medical folder to the destination before the medical information exceeds the set capacity. It is made possible to suppress increase of transmission cost as compared with the case of transmitting the medical information every time the medical information is accumulated.

### (Others 1)

As described above, the medical information processing system of the present invention is a medical information processing system constructed to connect two or more medical information processing apparatuses one another via the Internet, to convert patient information into e-mail and to transmit the e-mail from one of the two or more medical information processing apparatuses to the other medical information processing apparatuses via the Internet, wherein the medical information processing apparatus on the transmission side includes data recording/reproducing means on the transmission side, data inputting means on the transmission side and data processing means on the transmission side, the data recording/reproducing means on the transmission side has a database for recording patient information, the data inputting means on the transmission side is provided to be able to input the patient information to the database of the data recording/reproducing means on the transmission side, and the data processing means on the transmission side is set to check the database based on a predetermined setting condition, to automatically prepare e-mail attached with new patient information in the database and a patient ID in the information, and to transmit the e-mail to one or more predetermined destination medical information processing apparatuses. Consequently, when there is new information in the database, this new information and the patient ID of the information can be converted into e-mail, and the e-mail can be automatically transmitted to the destination medical information processing apparatuses. Hence, in the case where the image data, the accompanying patient data and the like are to be shared or copied among a plurality of ophthalmologic image information systems by use of a network such as the Internet connected through a telephone line, an operation becomes unnecessary for an operator him/herself to call out such information from the database, and prepare and transmit e-mail having the information attached thereto at each sharing and copying. This prevents occurrence of the problems such as a mistake in designating the destination or the information to be attached to the e-mail, enables necessary new information to be transmitted to the destinations simply, automatically and securely without an error, and enables update of the information (data) in the database of the destinations. In addition, also when adding graphic information and observation/diagnosis information to the images sent by e-mail and sending back the images to the transmitter, it is not necessary to designate the destination to be replied to by separately referring to an address book and the like based on the patient information of the images at each sending back as has been done heretofore. This simplifies the procedure for the designation, causes no mistake in designating the destination to be replied to, and prevents the destination itself to be replied from being mistaken.

Moreover, the new information in the database is any one of new data newly added, changed data and partially deleted information. Therefore, when there is new data, changed data or partially deleted information in the database, such information and the patient ID of the information can be automatically converted into e-mail, and the e-mail can be automatically transmitted to the destination medical information processing apparatuses. Specifically, when there is new text data or new image data newly added and stored in the database, the new text data or the new image data can be automatically converted into the e-mail, and the e-mail can be automatically transmitted to the destination medical information processing apparatuses. Also in this case, in the case where the image data, the accompanying patient data and the like are to be shared or copied among a plurality of ophthalmologic image information systems by use of a network such as the Internet connected through a telephone line, an operation becomes unnecessary for an operator him/herself to call out such information from the database, and prepare and transmit e-mail having the information attached thereto at each sharing and copying. This prevents occurrence of the problems such as a mistake in the destination or the information to be attached to the e-mail, enables necessary new information to be transmitted to the destination simply, automatically and securely without an error, and enables update of the information (data) in the databases of the destinations.

Furthermore, the setting condition is any one of a specified time interval, a previously specified time and activation through some explicit linkage. Therefore, the database is checked at the specified time intervals, at the previously specified time or at the activation through some explicit linkage, and if there is new text data or new image data newly added and stored in the database, e-mail attached with the new text data or the new image data and the accompanying patient data is automatically prepared, and transmitted to the one or more predetermined destination medical information processing apparatuses. Therefore, the information (data) in the database of the destination can be updated at the specified time intervals or at the previously specified time.

Here, as the activation through some explicit linkage, for example, conceived is a module for checking the set capacity of transmittable data (checking software or checking program) to be described later or the like.

Moreover, in the case where the setting condition is a set capacity of transmittable data of the e-mail, occurrence of a situation such that a transmission data capacity becomes too large to be transmitted can be previously prevented. Since a constitution is adopted, in which the set capacity can be set based on a mailbox having a smaller capacity between mailboxes on the transmission side and the receiving side, occurrence of a situation such that a transmission/receiving data capacity on the transmission side or the receiving side becomes too large to transmit can be previously prevented.

Furthermore, in the case where the data processing means on the transmission side is set to prepare a transmission message file to the effect that the e-mail has been transmitted when automatically preparing the e-mail, and to transmit the transmission message file to the one or more predetermined destination medical information processing apparatuses and facsimiles placed in the places where the medical information processing apparatuses are located via a transmission route different from the transmission route of the e-mail, even if trouble occurs in one of the transmission routes, confirmation is made on the destination side (receiving side) that the e-mail attached with the new text data or the new image data and the accompanying patient data has not been delivered thereto, and retransmission of the e-mail can be requested to the transmission side rapidly.

Moreover, in the case where the data processing means on the transmission side is set to prepare a list of transmission message files and to transmit the list to the one or more predetermined destination medical information processing apparatuses and the facsimiles placed in the places where the medical information processing apparatuses are located via the transmission route different from the transmission route of the e-mail at specified time intervals or at a previously specified time, even if a trouble occurs in one of the transmission routes, confirmation is made on the destination side (receiving side) that the e-mail attached with the new text data or the new image data and the accompanying patient data has not been delivered thereto at each set time or each specified time interval, and retransmission of the e-mail can be requested to the transmission side rapidly. In this case, even if trouble occurs in both of the transmission routes, since the transmission message file has not been delivered, the occurrence of a trouble at least in the route for the transmission message file between the transmission routes can be known.

Furthermore, the data processing means on the transmission side can be set to prepare the list of the transmission message files and to transmit the list to the one or more predetermined destination medical information processing apparatuses via the same transmission route as transmission route of the e-mail at specified time intervals or at a previously specified time. In this case, the medical information processing apparatus on the destination side transmits reply mail to the effect that the list of the transmission message files has been received to the transmission side in the case of receiving the list of the transmission message file.

### (Others 2)

As described above, by disposing the medical information processing terminals 55b, 56b and 57b in buildings (hospitals, center or house) where specialists with knowledge and rich experience concerning a specific disease stay, the specialists can grade the disease from the medical inspection information without being sent to a plurality of hospitals and the diabetes center 50 for the specific disease, and can send a grading report to the diabetes center 50. Thus, waste of traveling time required when the specialists are sent to the plurality of hospitals and the diabetes center 50 for the specific disease is avoided, and the grading of the disease by the specialists with rich experience and knowledge is performed rapidly, accurately and efficiently, thus enabling the grading for the medical inspection information of more patients. In addition, since only the grading report is transmitted to the host computer 51, transmission time to the host computer 51 is short, and the medical inspection information is not recorded in plural in the host computer 51.

Moreover, in the case where the automatic information transmission/receiving software 63 or 64 of the host computer 51 is provided to be capable of transmitting the medical inspection information to one of the plurality of medical information processing terminals 55b, 56b and 57b, and provided to be capable of sequentially transmitting the medical inspection information received by the one of the medical information processing terminals 55b, 56b and 57b together with the grading report to the rest of the medical information processing terminals 55b, 56b and 57b; and, among the medical information processing terminals 55b, 56b and 57b, the medical information processing terminal having received the medical inspection information and the grading report finally transmits only the grading report to the host computer 51, the host computer 51 have to transmit the medical inspection information to only one of the medical information processing terminals 55b, 56b and 57b. Therefore, processing in the host computer 51 is simplified.

Furthermore, in the case where the medical inspection information is simultaneously transmitted to the plurality of medical information processing terminals 55b, 56b and 57b from the host computer 51, and the grading reports are simultaneously transmitted from the plurality of medical information processing terminals 55b, 56b and 57b to the host computer 51, preparation of the grading reports by the plurality of specialists based on the medical inspection information can be simultaneously progressed. Consequently, the host computer 51 can obtain the grading reports prepared by each specialist in a short time, and the final comprehensive grading by integrating the grading reports prepared by the specialists can be performed rapidly.

Moreover, in the case where each of the medical information processing terminals 55b, 56b and 57b includes the grading image display software 61 for displaying the medical images as grading images, a mouse as designating means for designating a specific portion or a specific range on the medical image displayed by the grading image display software 61, and the cursor manipulated by the mouse; and in addition to the grading report, the information of the specific portion or the specific range on the medical image, which is designated by the designating means, is set to be transmitted to the host computer 51, confirmation can also be made in the host computer 51 (diabetes center side) as to which portion of the medical image the grading of the disease symptoms by each specialist is based on.

Furthermore, in the case where the information of the specific portion or the specific range is partial image information as compared with the case of transmitting the medical image used for the grading, where the specific portion or the specific range is marked, and the grading report to the medical information processing apparatus on the center side, a transmission time of the grading report including the partial image information to the medical information processing apparatus on the center side can be shortened.

Moreover, in the case where the information of the specific portion or the specific range is coordinate information of the specific portion or the specific range on the medical image designated by the designating means, as compared with the case of transmitting the medical image used for the grading, where the specific portion or the specific range is marked, and the grading report to the host computer 51 (diabetes center), a transmission time of the grading report including the partial image information to the host computer 51 (diabetes center) can be shorted. In addition, in this case, since the image information is not included at all, as compared with the case of transmitting the grading report including the partial image to the host computer 51 (diabetes center), the transmission time can be further shortened.

### (Others 3)

Furthermore, in the system including two or more information processing apparatuses connected one another via the Internet, attaching the transmission information correlated with the individual information composed of the ID and the specific information to e-mail, and transmitting the e-mail from one of the two or more information processing apparatuses to the other information processing apparatuses via the Internet, the information processing apparatus on the transmission side can be set to automatically prepare e-mail attached with only the ID obtained by excluding the individual information from the transmission information and the specific information, and to transmit the e-mail to the one or more predetermined destination medical information processing apparatuses. In this case, the individual information may be personal information. Moreover, as described above, this ID can be set as a patient ID in the hospital, the specific information can be set as a patient information, and the personal information can be set as information in the patient information other than the ID. Moreover, as described above, the ID can be converted by random numbers, attached to the e-mail and transmitted. As described above, the ID and the transmission information, excluding the individual information, are transmitted via the Internet. Thus, even if the individual information (personal information) can be known from the ID and the transmission information on the transmission side, a relation between the individual information (personal information) and the transmission information is not grasped on the Internet and the destination side. Therefore, even if the transmission information such as medical information is seen by others on the Internet and the destination side, protection of the individual information can be securely achieved.

### Effects of the Invention

As described above, the invention of claim 1 is a medical information processing system for connecting two or more medical information processing apparatuses one another via the Internet, converting patient information into e-mail and transmitting the e-mail from one of the two or more medical information processing apparatuses to the other medical information processing apparatuses via the Internet, which is constituted that the medical information processing apparatus on a transmission side includes data recording/reproducing means on the transmission side, data inputting means on the transmission side and data processing means on the transmission side, the data recording/reproducing means on the transmission side has a database for recording patient information, the data inputting means on the transmission side is provided to be able to input the patient information to the database of the data recording/reproducing means on the transmission side, and the data processing means on the transmission side is set to check the database based on a predetermined setting condition, to automatically prepare e-mail, attached with new patient information in the database and a patient ID in the information, and to transmit the e-mail to one or more predetermined destination medical information processing apparatuses. Therefore, when there is new information in the database, the new information and the patient ID in the information can be automatically converted into e-mail, and the e-mail can be automatically transmitted to the destination medical information processing apparatuses.

Hence, in the case where the image data, the accompanying patient data and the like are to be shared or copied among a plurality of ophthalmologic image information systems by use of a network such as the Internet connected through a telephone line, eliminated is necessity for an operator him/herself to call out such information from the database, and to prepare and transmit e-mail having the information attached thereto at each sending back. This prevents occurrence of the problems such as mistaking the destination or the information to be attached to the e-mail, enables necessary new information to be transmitted to the destinations simply, automatically and securely without an error, and enables an update of the information (data) in the database of the destinations. In addition, also when adding graphic information and observation/diagnosis information to the images sent by e-mail and sending back the images to the transmitter, it is not necessary to designate the destination to be replied to by separately referring to an address book and the like based on the patient information of the images at each sending back as in a conventional way. This simplifies the procedure for the designation, causes no mistake in designating the destination to be replied, and prevents the destination to be replied itself from being mistaken.

Moreover, the invention of claim 2 is constituted in such a manner that the new information in the database is new data newly added, changed data or partially deleted information. Therefore, when there is new data, changed data or partially deleted information in the database, such information and the patient ID of the information can be automatically converted into e-mail, and the e-mail can be automatically transmitted to the destination medical information processing apparatuses.

Specifically, when there is new text data or new image data newly added and stored in the database, the new text data or the new image data can be automatically converted into the e-mail, and the e-mail can be automatically transmitted to the destination medical information processing apparatuses. Also in this case, in the case where the image data, the accompanying patient data and the like are to be shared or copied among a plurality of ophthalmologic image information systems by use of a network such as the Internet connected through a telephone line, an operation, in which an operator him/herself calls out such information from the database and prepares and transmits e-mail having the information attached thereto at each sharing and copying, becomes unnecessary. This previously prevents occurrence of the problems such as a mistake about the destination or the information to be attached to the e-mail, enables necessary new information to be transmitted to the destination simply, automatically and securely without an error, and enables update of the information (data) in the database of the destinations.

Furthermore, in the invention of claim 3, the setting condition is a specified time interval, a previously specified time or activation through some explicit linkage. Therefore, the database is checked at the specified time intervals, at the previously specified time or at the activation through some explicit linkage, and if there is new text data or new image data newly added and stored in the database, e-mail attached with the new text data or the new image data and the accompanying patient data is automatically prepared, and transmitted to the one or more predetermined destination medical information processing apparatuses. Therefore, the information (data) in the database of the destination can be updated at the specified time intervals or at the previously specified time.

The invention of claim 4 is constituted in such a manner that the setting condition is a set capacity of data transmittable as the e-mail. Therefore, occurrence of a situation such that a transmission data capacity becomes too large to transmit can be previously prevented.

The invention of claim 5 is constituted in such a manner that the set capacity can be set based on a mailbox having a smaller capacity between mailboxes on the transmission side and the receiving side. Therefore, occurrence of a situation such that the capacity of transmitted data or received data on the transmission side or the receiving side becomes too large to transmit can be previously prevented.

The invention of claim 6 is constituted in such a manner that the data processing means on the transmission side is set to prepare a transmission message file to the effect that the e-mail has been transmitted when automatically preparing the e-mail and to transmit the transmission message file to the one or more predetermined destination medical information processing apparatuses and the facsimiles placed in the places where the medical information processing apparatuses are located via a transmission route different from a transmission route of the e-mail. Therefore, even if trouble occurs in one of the transmission routes, confirmation is made on the destination side (receiving side) that the e-mail attached with the new text data or the new image data and the accompanying patient data has not been delivered thereto, and retransmission can be rapidly requested to the transmission side.

The invention according to claim 7 is constituted in such a manner that the data processing means on the transmission side is set to prepare a list of transmission message files and to transmit the list to the one or more predetermined destination medical information processing apparatuses and the facsimiles placed in the places where the medical information processing apparatuses are located via the transmission route different from the transmission route of the e-mail at specified time intervals or at a previously specified time. Therefore, even if trouble occurs in one of the transmission routes, confirmation is made on the destination side (receiving side) that the e-mail attached with the new text data or the new image data and the accompanying patient data has not been delivered thereto at each set time or each specified time interval, and retransmission can be rapidly requested to the transmission side. In this case, even if trouble occurs in both of the transmission routes, since the transmission message file has not been delivered, the occurrence of trouble at least in the route for the transmission message file between the transmission routes can be known.

The invention according to claim 10 serves as a recording medium for an information processing system, wherein programs for the medical information processing systems of claims 1 to 7 are stored. Therefore, when there is new information in the database, the new information and the patient ID of the information can be automatically converted into e-mail, and the e-mail can be automatically transmitted to the destination medical information processing apparatuses by use of the program in the recording medium. Moreover, effects of claims 1 to 7 can also be expected by use of this recording medium.

## Claims

1. A medical information processing system comprising: two or more medical information processing apparatuses connected to the Internet for transmitting patient information from one of the two or more medical information processing apparatuses to the other medical information processing apparatuses by e-mail via the Internet,
wherein said medical information processing apparatus on a transmission side includes data recording/reproducing means on the transmission side, data inputting means on the transmission side and data processing means on the transmission side,
said data recording/reproducing means on the transmission side has a database for recording patient information,
said data inputting means on the transmission side is provided to be able to input the patient information to the database of the data recording/reproducing means on the transmission side, and
said data processing means on the transmission side is set to check the database based on a predetermined setting condition, to automatically prepare e-mail attached with new patient information in the database and a patient ID in the information, and to transmit the e-mail to one or more predetermined destination medical information processing apparatuses.

2. A medical information processing system according to claim 1, wherein the new information in the database is any one of new data newly added, changed data and partially deleted information.

3. A medical information processing system according to claim 1, wherein the setting condition is any one of a specified time interval and a previously specified time.

4. A medical information processing system according to any one of claims 1 and 2, wherein the setting condition is a set capacity of data transmittable as the e-mail.

5. A medical information processing system according to claim 4, wherein the set capacity can be set based on a mailbox having a smaller capacity between mailboxes on the transmission side and the receiving side.

6. A medical information processing system according to claim 1, wherein said data processing means on the transmission side is set to prepare a transmission message file to the effect that the e-mail has been transmitted when automatically preparing the e-mail, and to transmit the transmission message file to any one of the one or more predetermined destination medical information processing apparatuses and facsimiles placed in places where the medical information processing apparatuses are located via a transmission route different from a transmission route of the e-mail.

7. A medical information processing system according to claim 6, wherein the data processing means on the transmission side is set to prepare a list of transmission message files and to transmit the list to any of the one or more predetermined destination medical information processing apparatuses and the facsimiles placed in the places where the medical information processing apparatuses are located via the transmission route different from the transmission route of the e-mail at any of a certain specified time interval and a previously specified time.

8. A medical information processing system according to claim 1, wherein the data processing means on the transmission side is set to prepare a list of transmission message files and to transmit the list to the one or more predetermined destination medical information processing apparatuses via the same transmission route as a transmission route of the e-mail at any of a certain specified time interval and a previously specified time.

9. A medical information processing system according to claim 8, wherein the medical information processing apparatus on a destination side is set to transmit reply mail to the effect that the list of the transmission message files has been received when receiving the list.

10. A recording medium for an information processing system, wherein programs for the medical information processing systems as stated in claims 1 to 7 are stored.
